# EUROPEAN PATENT SPECIFICATION

(11) **EP 2 935 592 B1**
(45) Date of publication and mention of the grant of the patent: **21.03.2018**
(21) Application number: 13821307.9
(22) Date of filing: 19.12.2013
(51) Int. Cl.: C12N 15/82

(54) **IMPROVED SPLIT SEED SOYBEAN TRANSFORMATION FOR EFFICIENT AND HIGH-THROUGHPUT TRANSGENIC EVENT PRODUCTION**
VERBESSERTE TRANSFORMATION VON SOJABOHNEN MIT GETEILTEM SAMEN ZUR EFFIZIENTEN HERSTELLUNG TRANSGENER EREIGNISSE MIT HOHEM DURCHSATZ
TRANSFORMATION DE SOJA À GRAINE FENDUE AMÉLIORÉE POUR LA PRODUCTION D'ÉVÉNEMENT TRANSGÉNIQUE EFFICACE ET À RENDEMENT ÉLEVÉ

(30) Priority: 19.12.2012 US 201261739349 P
(43) Date of publication of application: 28.10.2015
(73) Proprietor: Dow AgroSciences LLC, Indianapolis, IN 46268 (US)
(72) Inventor: PAREDDY, Dayakar, Carmel, Indiana 46033 (US); CHENNAREDDY, Sivarama Reddy, West Lafayette, Indiana 47906 (US); MINNICKS, Tatyana, Indianapolis, Indiana 46268 (US); KARPOVA, Olga, Indianapolis, Indiana 46268 (US); GRIFFIN, David, Indianapolis, Indiana 46268 (US); JAYAKUMAR, Pon Samuel, Carmel, Indiana 46032 (US); SMITH, Kelly, Indianapolis, Indiana 46268 (US); SARRIA-MILAN, Rodrigo, West Lafayette, Indiana 47906 (US)
(74) Representative: f & e patent
(86) International application number: PCT/US2013/076540
(87) International publication number: WO 2014/100406

(56) References cited:
- US-B1- 7 473 822
- MARGIE M PAZ ET AL: "Improved cotyledonary node method using an alternative explant derived from mature seed for efficient Agrobacterium-mediated soybean transformation", PLANT CELL REPORTS, SPRINGER, BERLIN, DE, vol. 25, no. 3, 1 March 2006 (2006-03-01), pages 206-213, XP019335551, ISSN: 1432-203X, DOI: 10.1007/S00299-005-0048-7

## Description

### FIELD OF THE DISCLOSURE

The disclosure relates generally to methods of producing transgenic soybean. Methods are provided for transformation of soybean. Methods of the disclosure are useful for efficient and high throughput transgenic production of soybean and commercial development of transgenic soybean products.

### BACKGROUND

Soybean (*Glycine max*) is one of the most important agricultural crops, with an annual crop yield of more than 200 million metric tons, and an estimated value exceeding 40 billion U.S. dollars worldwide. Soybean accounts for over 97% of all oilseed production globally. Thus, reliable and efficient methods for improving the quality and yield of this valuable crop are of significant interest.

Traditional breeding methods for improving soybean have been constrained because the majority of soybean cultivars are derived from only a few parental lines, leading to a narrow germplasm base for breeding. Christou et al., TIBTECH 8:145-151 (1990). Modem research efforts have focused on plant genetic engineering techniques to improve soybean production. Transgenic methods are designed to introduce desired genes into the heritable germline of crop plants to generate elite plant lines. The approach has successfully increased the resistance of several other crop plants to disease, insects, and herbicides, while improving nutritional value.

Several methods have been developed for transferring genes into plant tissue, including high velocity microprojection, microinjection, electroporation, and direct DNA uptake. *Agrobacterium-mediated* gene transformation has more recently been used to introduce genes of interest into soybeans. However, soybeans have proven to be a challenging system for transgenic engineering. Efficient transformation and regeneration of soybean explants is difficult to achieve, and frequently hard to repeat.

*Agrobacterium tumefaciens,* a pathogenic, soil-dwelling bacterium, has the inherent ability to transfer its DNA, called T-DNA, into host plant cells and to induce the host cells to produce metabolites useful for bacterial nutrition. Using recombinant techniques, some or all of the T-DNA may be replaced with a gene or genes of interest, creating a bacterial vector useful for transforming the host plant. *Agrobacterium-mediated* gene transfer is typically directed at undifferentiated cells in tissue culture, but may also be directed at differentiated cells taken from the leaf or stem of the plant. A number of procedures have been developed for *Agrobacterium-*mediated transformation of soybean, which may loosely be classified based on the explant tissue subjected to transformation.

U.S. Pat. No. 7,696,408, Olhoft, *et* a/*.,* discloses a cotyledonary node method for transforming both monocotyledonous and dicotyledonous plants. The "cot node" method involves removing the hypocotyl from 5-7 day old soybean seedlings by cutting just below the cotyledonary node, splitting and separating the remaining hypocotyl segment with the cotyledons, and removing the epicotyl from the cotyledon. The cotyledonary explant is wounded in the region of the axillary bud and/or cotyledonary node, and cultivated with *Agrobacterium tumefaciens* for five days in the dark. The method requires in-vitro germination of the seeds, and the wounding step introduces significant variability.

U.S. Pat. No. 6,384,301, Martinelli et al., discloses *Agrobacterium*-mediated gene delivery into living meristem tissue from soybean embryos excised from soybean seeds, followed by culturing of the meristem explant with a selection agent and hormone to induce shoot formation. Like the "cot node" method, the meristem explants are preferably wounded prior to infection.

U.S. Pat. No. 7,473,822, Paz *et* a/., discloses a modified cotyledonary node method called the "half-seed explant" method. Mature soybean seeds are imbibed, surface-sterilized and split along the hilum. Prior to infection, the embryonic axis and shoots are completely removed, but no other wounding occurs. *Agrobacterium-mediated* transformation proceeds, potential transformants are selected, and explants are regenerated on selection medium.

Transformation efficiencies remain relatively low with these methods, on the order of 0.3% to 2.8% for the "cot node" method, 1.2 to 4.7% for the "meristem explant" method, and between 3.2% and 8.7% (overall 4.9%) for the "half-seed explant" method. Transformation efficiencies of approximately 3% are typical in the art.

An improved "split-seed" transgenic protocol may accelerate future production and development of transgenic soybean products. An efficient and high-throughput method for stable integration of a transgene into soybean tissue would facilitate breeding programs and have the potential to increase crop productivity.

### SUMMARY OF THE DISCLOSURE

The invention is defined in the claims. The present disclosure relates to a method of transforming plant cells. More particularly, the disclosure relates to a method of transforming soybean (*Glycine max*) using *Agrobacterium-mediated* transformation of a split soybean seed, wherein the split soybean seed retains a portion of the embryonic axis.

In an embodiment, the present disclosure relates to a method of producing a transgenic event, that includes inserting a cutting tool into a seed coat of a soybean seed to form a first cotyledon segment and a second cotyledon segment, inoculating the first cotyledon segment including a 1/4, 1/3, 1/2, 2/3, 3/4 or the full length portion of the embryonic axis with *Agrobacterium,* the *Agrobacterium* including at least one transgene, and culturing the inoculated cotyledon segment to produce a transgenic event in a transformed plant cell.

In another embodiment, the present disclosure relates to a method of producing a transgenic event, that includes inserting a cutting tool into a seed coat of a soybean seed to form a cotyledon segment including a 1/4, 1/3, 1/2, 2/3, 3/4 or the full length portion of the embryonic axis, inoculating the cotyledon segment including the portion of the embryonic axis with *Agrobacterium,* the *Agrobacterium* including at least one transgene, culturing the inoculated cotyledon segment to produce a transgenic event in a transformed plant cell, and producing a whole, fertile plant from the cultured inoculated cotyledon segment, the whole, fertile plant including the transgenic event.

### BRIEF DESCRIPTION OF THE FIGURES

**FIG. 1** is a plan view of a soybean seed.
**FIG. 2** is a side elevation view of the soybean seed of FIG. 1.
**FIG. 3** is a cross-sectional elevation view of the soybean seed taken along the line 3-3 in FIG. 1.
**FIG. 4** is a view similar to FIG. 3 showing a cutting tool engaged with a portion of the embryonic axis of the soybean seed.
**FIG. 5** is a cross-sectional elevation view of the soybean seed taken along the line 5-5 in FIG. 1 showing a cutting tool inserted into the soybean along the soybean's longitudinal axis.
**FIG. 6** is a plan view of a pair of cotyledon segments.
**FIG. 7** shows a plasmid map of pDAB9381, a construct that may be used in the *Agrobacterium-mediated* transformation of soybean explants, according to a particular embodiment.
**FIG. 8** shows a plasmid map of pDAB107533.
**FIG. 9** shows a graph depicting the percentage of explants exhibiting yellowing on at least half of the cotyledon for the varying rates of glyphosate is provided.
**FIG. 10** shows a plasmid map of pDAB105958.

### SEQUENCE LISTING

The nucleic acid sequences listed in the accompanying sequence listing are shown. Only one strand of each nucleic acid sequence is shown, but the complementary strand is understood as being included by any reference to the displayed strand. In the accompanying sequence listing:
**SEQ ID NO:1** shows the YFP Plant Transcription Unit (PTU) in the plasmid construct pDAB9381, for use in the present disclosure.
**SEQ ID NO:2** shows the PAT Plant Transcription Unit (PTU) in the plasmid construct pDAB9381, for use in the present disclosure.
**SEQ ID NO:3** shows the DGT-28 Plant Transcription Unit (PTU) in the plasmid construct pDAB107553, for use in the present disclosure.
**SEQ ID NO:4** shows the PAT Plant Transcription Unit (PTU) in the plasmid construct pDAB107553, for use in the present disclosure.
**SEQ ID NO:5** shows the HPT Plant Transcription Unit (PTU) in the plasmid construct pDAB105958, for use in the present disclosure.
**SEQ ID NO:6** shows the PAT Plant Transcription Unit (PTU) in the plasmid construct pDAB105958, for use in the present disclosure.

### DETAILED DESCRIPTION

The invention is defined in the claims. Disclosed herein are methods for the efficient and high-throughput transformation of soybean. The deployment of the disclosed soybean transformation method results in transformation frequencies that are significantly improved over previous known methods, and results in transformation frequencies of up to 20.3%. The novel soybean transformation system is about 3 to 8 fold more efficient than other methods, *i.e*., the "cot node" method and "half-seed explant" method, and serves as a foundation to improve the commercial development of transgenic soybean plants.

Generally, embodiments of the disclosure relate to a novel method for transformation of split soybean seeds comprising a portion of an embryo axis. The novel method is an improvement of other known transformation methods, and results in the efficient production of transgenic soybean plants.

In an embodiment, a novel method for transforming soybeans with a transgene is disclosed. Embodiments of this method include splitting a soybean seed longitudinally to obtain a split soybean seed, wherein a 1/4, 1/3, 1/2, 2/3, 3/4 or the full length portion of an embryonic axis remains attached to the split soybean seed. Next, the split soybean seed comprising said portion of the embryonic axis is transformed with at least one transgene using a transformation method. Soybean transformants are selected from the transformed split soybean seed comprising a portion of the embryonic axis.

In one embodiment of the subject disclosure, the soybean seed is imbibed before splitting the soybean seed. The soybean seed may be imbibed from 14 to 16 hours. In addition, the soybean seed may be imbibed for other alternative periods of time. For example, the soybean seed may be imbibed for 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, or 48 hours of time.

In a second embodiment of the subject disclosure, the soybean seed is split longitudinally along the hilum of the soybean seed.

In an embodiment, the soybean seed is split so that the embryonic axis remains attached to the nodal end of the soybean seed. The embryonic axis which is retained with the soybean seed can comprise any portion of the embryonic axis seed structure. As such, a 1/4, 1/3, 1/2, 2/3, 3/4 or the full length portion or amount of embryonic axis which is retained while splitting soybean the seed is within the scope of the disclosure. According to the invention ¼, ⅓, ½, 2/3, ¾, or the entire embryonic axis may be retained when splitting the soybean seed.

In a further embodiment, the split soybean seed with the retained portion of embryonic axis are transformed by inoculating the split soybean seed with a strain of *Agrobacterium tumefaciens* harboring one or more transgenes within a construct. Other transformation methods are known in the art. Common methods are e.g. a high velocity microprojection-mediated transformation method, a microinjection-mediated transformation method, an electroporation-mediated transformation method, or a direct DNA uptake-mediated transformation method, or any other plant transformation method.

In an embodiment of the subject disclosure, the split soybean seed with the retained portion of embryonic axis is transformed, thereby introducing a transgene into a soybean cell. The introduction of the transgene within the soybean cell may result in a stably or transiently transformed soybean plant cell. In an aspect of this embodiment, one or more transgenes can be transformed into the soybean plant cell. In a further embodiment, the transgene comprises a promoter, an open reading frame, and a 3' untranslated region. The open reading frame can encode a gene, wherein the gene is a marker gene, an agronomic gene, or any other type of gene.

In an embodiment, the transformed split soybean seed with the retained portion of an embryonic axis is selected for using an antimicrobial selection agent. Examples of antimicrobial selection agents include, but are not limited to, glufosinate, phosphinothricin, 2,4-D, kanamycin, and glyphosate.

Embodiments of the present disclosure relate to regenerating one or more explants from the split soybean seed comprising a portion of the embryonic axis. The regeneration of the explant is typically made on selection medium, but can be made on other types of media known in the art. Upon, successfully regenerating an explant, the formation of one or more shoots can be induced by tissue culture (e.g. "culturing") on appropriate medium. As a further embodiment of the disclosure, the one or more shoots can be cultivated into a whole, fertile, soybean plant. Said shoots can comprise transformed germline cells.

It is known in the art that the whole, fertile, mature soybean plant may be sexually crossed with another soybean plant to generate a soybean progeny plant. The explants produced from transforming the split soybean seeds comprising the attached portion of embryonic axis are isolated and advanced to cell-tissue medium for shoot induction (two weeks without selection and two weeks with selection), shoot elongation (with selection), and rooting (without selection). The advancement of the transgenic soybean explants through these stages of cell culture result in transgenic plants. The transgenic plants are confirmed to contain a transgene that is stably integrated within the soybean genome via molecular analysis. Specific transgenic soybean plants are then grown to maturity in the greenhouse. In experiments, 45% of T1 progeny, similar to that of the cotyledonary method, were found to possess a stably integrated copy of the transgene which was heritable to progeny soybean plants. The heritability of the transgene to subsequent generations was confirmed by herbicide screening with the herbicide Liberty™ and by molecular analysis.

In another embodiment, the present disclosure relates to a method of producing a transgenic event, that includes inserting a cutting tool into a seed coat of a soybean seed to form a first cotyledon segment and a second cotyledon segment, inoculating the first cotyledon segment including a 1/4, 1/3, 1/2, 2/3, 3/4 or the full length portion of the embryonic axis with *Agrobacterium,* the *Agrobacterium* including at least one transgene, and culturing the inoculated cotyledon segment to produce a transgenic event in a transformed plant cell.

In an embodiment, the method includes removing the seed coat from the first cotyledon segment and the second cotyledon segment prior to inoculating the first cotyledon segment. In a further embodiment, the method includes inserting the cutting tool and cutting the embryonic axis longitudinally to retain a 1/4, 1/3, 1/2, 2/3, 3/4 or the full length portion of the embryonic axis with each cotyledon segment. In an aspect of the embodiment, the embryonic axis of the soybean seed has an uncut length prior to inserting the cutting tool, and the portion of the embryonic axis of the first cotyledon segment has a length that is about 1/3 to 1/2 of the uncut length of the embryonic axis. In yet another aspect of the embodiment, the portion of the embryonic axis of the first cotyledon segment is cut to retain a portion of the embryonic axis attached to the first cotyledon segment, and the retained portion of the embryonic axis has a length that is about 1/3 to 1/2 of the uncut length of the embryonic axis. In a further aspect of the embodiment, the embryonic axis of the soybean seed has an uncut length prior to inserting the cutting tool, and the portion of the embryonic axis of the first cotyledon segment has a length equal to the uncut length.

In another embodiment, inserting the cutting tool includes cutting the soybean seed longitudinally along a hilum of the soybean seed. In an aspect of the embodiment, the hilum on an outer surface of the soybean seed is located, and the cutting tool is oriented relative to the soybean seed to align the cutting tool with the hilum prior to inserting the cutting tool. In a further aspect of the embodiment, each cotyledon segment includes a portion of the hilum. In another aspect of the embodiment, the soybean seed has a longitudinal axis extending through the hilum, the longitudinal axis dividing the soybean seed into a first side and a second side. In a further aspect of the embodiment, the first side of the soybean seed is grasped prior to inserting the cutting tool.

In a subsequent embodiment, the present disclosure relates to inserting the cutting tool and advancing the cutting tool into the embryonic axis of the soybean seed prior to forming the first and second cotyledon segments. In an alternative embodiment, the first and second cotyledon segments are formed prior to inserting the cutting tool and advancing the cutting tool into the embryonic axis to produce a first cotyledon segment that retains a portion of the embryonic axis has a length that is a fraction (e.g., about 1/3 to 1/2) of the uncut length of the embryonic axis.

In a further embodiment, the method of the present disclosure includes producing a whole, fertile, soybean plant from the cultured inoculated cotyledon segment, the whole, fertile, soybean plant including the transgenic event. In an aspect of the embodiment, the transgenic event of the whole, fertile, soybean plant comprises the transgenic event.

In another embodiment, the transgene is selected from the group consisting of an insecticidal resistance gene, herbicide tolerance gene, nitrogen use efficiency gene, water use efficiency gene, nutritional quality gene, DNA binding gene, and selectable marker gene.

In yet another embodiment, the present disclosure provides a method of producing a transgenic event, that includes inserting a cutting tool into a soybean seed coat of a seed to form a cotyledon segment including a 1/4, 1/3, 1/2, 2/3, 3/4 or the full length portion of the embryonic axis, inoculating the cotyledon segment and the portion of the embryonic axis with *Agrobacterium,* the *Agrobacterium* including at least one transgene, culturing the inoculated cotyledon segment to produce a transgenic event in a transformed plant cell, and producing a whole, fertile plant from the cultured inoculated cotyledon segment. The whole, fertile plant includes the transgenic event.

In a further embodiment, the seed is a dicotyledonous seed.

In another embodiment, inserting the cutting tool into the seed coat of the seed forms a first cotyledon segment and a second cotyledon segment. Each cotyledon segment includes a portion of the embryonic axis.

In a further embodiment, the transgene comprises a *pat, dgt-28,* or *hpt* selectable marker gene.

Unless otherwise indicated, the terms "a" and "an" as used herein refer to at least one.

As used herein, the term "explant" refers to a piece of soybean tissue that is removed or isolated from a donor plant (*e.g.,* from a donor seed), cultured in vitro, and is capable of growth in a suitable media.

As used herein, a "cotyledon" may generally refer to an embryonic leaf or "primary leaf" of the embryo of a seed plant. A cotyledon is also referred to in the art as a "seed leaf." Dicotyledonous species, such as soybean, have two cotyledons. A cotyledon segment refers to any portion of a cotyledon, whether it be an entire or whole cotyledon or a fragment or partial portion of a cotyledon. The "cotyledonary node" refers to the point of attachment of the cotyledons to the embyro in the seed or seedling, and may generally refer to the tissue associated with that point of attachment.

As used herein, the term "grasping" refers to holding or seizing the soybean seed with a tool or by hand. Any subsequent mechanism or action that allows the soybean seed to be firmly clasped is considered within the scope of the term grasping.

As used herein, the term "cutting tool" refers to a tool for cutting, severing or shearing a seed, seed cotyledon, see coat, hilum, embryonic axis, or any other seed structure. The cutting tool may include scalpels, razor blades, kitchen knives, knives, kogatanas, gravers, sickles, chisels, planes, and the like. In some aspects the cutting tool may comprise a cutting means attached to a shank or handle. In other aspects the cutting tool may comprise only a single cutting means that is unattached to any other structural feature. A cutting tool may also include a laser or highly pressurized fluid suitable for dividing a seed structure into a first and second cotyledon segment.

As used herein, the term "seed coat" refers to an integument of the ovule that serves as a seed's protective coat. Seed coat may be described by the alternative descriptive terms of "testa" or "husk", in addition to other similar terms known in the art. Seed coats may contain hydrophobic substances such as suberin, cutin, lignin, callose, pectin, waxes, and insoluble products of phenolic oxidation. In legumes, like soybean, the testa contains a palisade layer of thick-walled macrosclereid cells, whose caps extend into a suberized sub-cuticle, with a waxy cuticle external to the thicker suberin layer.

As used herein, the "hypocotyl" is that portion of the plant embryo or seedling below the cotyledons and above the root or radicle (embyronic root). In the seed, the hypocotyl is found just below the cotyledonary node, and may also be referred to as the "hypocotyledonous stem" or the "embryonic stem." As used herein, the hypocotyl may refer to the location, as well as the tissue found therein. The "epicotyl" is that portion of the plant embryo or seedling above the cotyledons and below the first true leaves. In the seed, the epicotyl is found just above the cotyledonary node, and may variously be referred to as the "embryonic shoot" or "future shoot." As used herein, the epicotyl may refer to the location, as described, or the tissue found therein.

As used herein, the terms "embryonic axis" or "embryo axis" refer to the major portion of the embryo of the plant, and generally includes the epicotyl and hypocotyl

As used herein, the term "genetically modified" or "transgenic" plant refers to a plant cell, plant tissue, plant part, plant germplasm, or plant which comprises a preselected DNA sequence which is introduced into the genome of a plant cell, plant tissue, plant part, plant germplasm, or plant by transformation.

As used herein, the term "transgenic," "heterologous," "introduced," or "foreign" DNA or gene refer to a recombinant DNA sequence or gene that does not naturally occur in the genome of the plant that is the recipient of the recombinant DNA or gene, or that occurs in the recipient plant at a different location or association in the genome than in the untransformed plant.

As used herein, the term "plant" refers to either a whole plant, plant tissue, plant part, including pollen, seeds, or an embryo, plant germplasm, plant cell, or group of plants. The class of plants that can be used in the method of the invention is not limited to soybeans, but may generally include any plants that are amenable to transformation techniques, including both monocotyledonous and dicotyledonous plants.

As used herein the term "progeny" refers to any subsequent generation of plant, plant part, plant tissue, of plant seed that is produced or derived (either directly or remotely) from an initial transformant (TO) that includes a transgenic event. Progeny further includes any subsequent generation of plant, plant part, plant tissue, or plant seed (F1, F2, F3, F4, F5, BC1, BC2, BC3, BC4, BC5, etc.) that is produced by a cross between individual or specific lines of plants, via backcrossing, forward breeding, marker assisted breeding, or any other known breeding methodology which results in the introgression of the transgenic event of the initial transformant.

As used herein the term "transformation" refers to the transfer and integration of a nucleic acid or fragment into a host organism, resulting in genetically stable inheritance. Host organisms containing the transformed nucleic acid fragments are referred to as "transgenic" or "recombinant" or "transformed" organisms. Known methods of transformation include *Agrobacterium tumefaciens* or *Agrobacterium rhizogenes* mediated transformation, calcium phosphate transformation, polybrene transformation, protoplast fusion, electroporation, ultrasonic methods (e.g., sonoporation), liposome transformation, microinjection, naked DNA, plasmid vectors, viral vectors, biolistics (microparticle bombardment), silicon carbide WHISKERS™ mediated transformation, aerosol beaming, or PEG transformation as well as other possible methods.

With regard to the production of genetically modified plants, methods for the genetic engineering of plants are well known in the art. For instance, numerous methods for plant transformation have been developed, including biological and physical transformation protocols for dicotyledenous plants as well as monocotyledenous plants (*e.g.,* Goto-Fumiyuki et al., Nature Biotech 17:282-286 (1999); Miki et al., Methods in Plant Molecular Biology and Biotechnology, Glick, B. R. and Thompson, J. E. Eds., CRC Press, Inc., Boca Raton, pp. 67-88 (1993)). In addition, vectors and *in vitro* culture methods for plant cell or tissue transformation and regeneration of plants are available, for example, in Gruber et al., Methods in Plant Molecular Biology and Biotechnology, Glick, B. R. and Thompson, J. E. Eds., CRC Press, Inc., Boca Raton, pp. 89-119 (1993).

A large number of techniques are available for transforming DNA into a plant host cell. Those techniques include transformation with disarmed T-DNA using *Agrobacterium tumefaciens* or *Agrobacterium rhizogenes* as the transformation agent, calcium phosphate transfection, polybrene transformation, protoplast fusion, electroporation, ultrasonic methods (e.g., sonoporation), liposome transformation, microinjection, naked DNA, plasmid vectors, viral vectors, biolistics (microparticle bombardment), silicon carbide WHISKERS mediated transformation, aerosol beaming, or PEG as well as other possible methods.

For example, the DNA construct may be introduced directly into the genomic DNA of the plant cell using techniques such as electroporation and microinjection of plant cell protoplasts, or the DNA constructs can be introduced directly to plant tissue using biolistic methods, such as DNA particle bombardment (see, *e.g.,* Klein et al. (1987) Nature 327:70-73). Additional methods for plant cell transformation include microinjection via silicon carbide WHISKERS™ mediated DNA uptake (Kaeppler et al. (1990) Plant Cell Reporter 9:415-418). Alternatively, the DNA construct can be introduced into the plant cell via nanoparticle transformation (*see, e.g.,* US Patent Application No. 12/245,685).

Another known method of plant transformation is microprojectile-mediated transformation wherein DNA is carried on the surface of microprojectiles. In this method, the expression vector is introduced into plant tissues with a biolistic device that accelerates the microprojectiles to speeds sufficient to penetrate plant cell walls and membranes. Sanford et al., Part. Sci. Technol. 5:27 (1987), Sanford, J. C., Trends Biotech. 6:299 (1988), Sanford, J. C., Physiol. Plant 79:206 (1990), Klein et al., Biotechnology 10:268 (1992).

Alternatively, gene transfer and transformation methods include, but are not limited to, protoplast transformation through calcium chloride precipitation, polyethylene glycol (PEG)- or electroporation-mediated uptake of naked DNA (see Paszkowski et al. (1984) EMBO J 3:2717-2722, Potrykus et al. (1985) Molec. Gen. Genet. 199:169-177; Fromm et al. (1985) Proc. Nat. Acad. Sci. USA 82:5824-5828; and Shimamoto (1989) Nature 338:274-276) and electroporation of plant tissues (D'Halluin et al. (1992) Plant Cell 4:1495-1505).

A widely utilized method for introducing an expression vector into plants is based on the natural transformation system of *Agrobacterium.* Horsch et al., Science 227:1229 (1985). *A. tumefaciens* and A. *rhizogenes* are plant pathogenic soil bacteria known to be useful to genetically transform plant cells. The Ti and Ri plasmids of A. *tumefaciens* and *A*. *rhizogenes,* respectively, carry genes responsible for genetic transformation of the plant. Kado, C. I., Crit. Rev. Plant. Sci. 10:1 (1991). Descriptions of *Agrobacterium* vector systems and methods for *Agrobacterium*-mediated gene transfer are also available, for example, Gruber *et al*., *supra,* Miki *et al*., *supra,* Moloney et al., Plant Cell Reports 8:238 (1989), and U.S. Patent Nos. 4,940,838 and 5,464,763.

If *Agrobacterium* is used for the transformation, the DNA to be inserted should be cloned into special plasmids, namely either into an intermediate vector or into a binary vector. Intermediate vectors cannot replicate themselves in *Agrobacterium.* The intermediate vector can be transferred into *Agrobacterium tumefaciens* by means of a helper plasmid (conjugation). The Japan Tobacco Superbinary system is an example of such a system (reviewed by Komari et al. (2006) In: Methods in Molecular Biology (K. Wang, ed.) No. 343: Agrobacterium Protocols (2nd Edition, Vol. 1) HUMANA PRESS Inc., Totowa, NJ, pp.15-41; and Komori et al. (2007) Plant Physiol. 145:1155-1160). Binary vectors can replicate themselves both in *E. coli* and in *Agrobacterium.* They comprise a selection marker gene and a linker or polylinker which are framed by the right and left T-DNA border regions. They can be transformed directly into *Agrobacterium* (Holsters, 1978). The *Agrobacterium* used as host cell is to comprise a plasmid carrying a *vir* region. The Ti or Ri plasmid also comprises the *vir* region necessary for the transfer of the T-DNA. The *vir* region is necessary for the transfer of the T-DNA into the plant cell. Additional T-DNA may be contained.

The virulence functions of the *Agrobacterium tumefaciens* host will direct the insertion of a T-strand containing the construct and adjacent marker into the plant cell DNA when the cell is infected by the bacteria using a binary T DNA vector (Bevan (1984) Nuc. Acid Res. 12:8711-8721) or the co-cultivation procedure (Horsch et al. (1985) Science 227:1229-1231). Generally, the *Agrobacterium* transformation system is used to engineer dicotyledonous plants (Bevan et al. (1982) Ann. Rev. Genet 16:357-384; Rogers et al. (1986) Methods Enzymol. 118:627-641). The *Agrobacterium* transformation system may also be used to transform, as well as transfer, DNA to monocotyledonous plants and plant cells. *See* U.S. Patent No. 5, 591,616; Hernalsteen et al. (1984) EMBO J 3:3039-3041; Hooykass-Van Slogteren et al. (1984) Nature 311:763-764; Grimsley et al. (1987) Nature 325:1677-179; Boulton et al. (1989) Plant Mol. Biol. 12:31-40; and Gould et al. (1991) Plant Physiol. 95:426-434.

Following the introduction of the genetic construct into plant cells, plant cells can be grown and upon emergence of differentiating tissue such as shoots and roots, mature plants can be generated. In some embodiments, a plurality of plants can be generated. Methodologies for regenerating plants are known to those of ordinary skill in the art and can be found, for example, in: Plant Cell and Tissue Culture, 1994, Vasil and Thorpe Eds. Kluwer Academic Publishers and in: Plant Cell Culture Protocols (Methods in Molecular Biology 111, 1999 Hall Eds Humana Press). The genetically modified plant described herein can be cultured in a fermentation medium or grown in a suitable medium such as soil. In some embodiments, a suitable growth medium for higher plants can include any growth medium for plants, including, but not limited to, soil, sand, any other particulate media that support root growth (*e.g.,* vermiculite, perlite, *etc.*) or hydroponic culture, as well as suitable light, water and nutritional supplements which optimize the growth of the higher plant.

Transformed plant cells which are produced by any of the above transformation techniques can be cultured to regenerate a whole plant which possesses the transformed genotype and thus the desired phenotype. Such regeneration techniques rely on manipulation of certain phytohormones in a tissue culture growth medium, typically relying on a biocide and/or herbicide marker which has been introduced together with the desired nucleotide sequences. Plant regeneration from cultured protoplasts is described in Evans, et al., "Protoplasts Isolation and Culture" in Handbook of Plant Cell Culture, pp. 124-176, Macmillian Publishing Company, New York, 1983; and Binding, Regeneration of Plants, Plant Protoplasts, pp. 21-73, CRC Press, Boca Raton, 1985. Regeneration can also be obtained from plant callus, explants, organs, pollens, embryos or parts thereof. Such regeneration techniques are described generally in Klee et al. (1987) Ann. Rev. of Plant Phys. 38:467-486.

Unless otherwise specifically defined, all technical and scientific terms used herein have the same meaning as commonly understood by those of ordinary skill in the art to which this disclosure belongs. Definitions of common terms in molecular biology can be found in, for example, Lewin B., Genes V, Oxford University Press, 1994 (ISBN 0-19-854287-9); Kendrew et al. (eds.), The Encyclopedia of Molecular Biology, Blackwell Science Ltd., 1994 (ISBN 0-632-02182-9); and Meyers R.A. (ed.), Molecular Biology and Biotechnology: A Comprehensive Desk Reference, VCH Publishers, Inc., 1995 (ISBN 1-56081-569-8).

It will be appreciated by one skilled in the art that the use of reporter or marker genes for selection of transformed cells or tissues or plant parts or plants can be included in the transformation vectors or construct. Examples of selectable markers include those that confer resistance to anti-metabolites such as herbicides or antibiotics, for example, dihydrofolate reductase, which confers resistance to methotrexate (Reiss, Plant Physiol. (Life Sci. Adv.) 13:143-149, 1994; see also Herrera Estrella et al., Nature 303:209-213, 1983; Meijer et al., Plant Mol. Biol. 16:807-820, 1991); neomycin phosphotransferase, which confers resistance to the aminoglycosides neomycin, kanamycin and paromycin (Herrera-Estrella, EMBO J. 2:987-995, 1983 and Fraley et al. Proc. Natl. Acad. Sci USA 80:4803 (1983)) and hygromycin phosphotransferase, which confers resistance to hygromycin (Marsh, Gene 32:481-485, 1984; see also Waldron et al., Plant Mol. Biol. 5:103-108, 1985; Zhijian et al., Plant Science 108:219-227, 1995); trpB, which allows cells to utilize indole in place of tryptophan; hisD, which allows cells to utilize histinol in place of histidine (Hartman, Proc. Natl. Acad. Sci., USA 85:8047, 1988); mannose-6-phosphate isomerase which allows cells to utilize mannose (WO 94/20627); ornithine decarboxylase, which confers resistance to the ornithine decarboxylase inhibitor, 2-(difluoromethyl)-DL-ornithine (DFMO; McConlogue, 1987, In: Current Communications in Molecular Biology, Cold Spring Harbor Laboratory ed.); and deaminase from *Aspergillus terreus,* which confers resistance to Blasticidin S (Tamura, Biosci. Biotechnol. Biochem. 59:2336-2338, 1995).

Additional selectable markers include, for example, a mutant acetolactate synthase, which confers imidazolinone or sulfonylurea resistance (Lee et al., EMBO J. 7:1241-1248, 1988), a mutant psbA, which confers resistance to atrazine (Smeda et al., Plant Physiol. 103:911-917, 1993), or a mutant protoporphyrinogen oxidase (see U.S. Pat. No. 5, 767, 373), or other markers conferring resistance to an herbicide such as glufosinate. Examples of suitable selectable marker genes include, but are not limited to: genes encoding resistance to chloramphenicol (Herrera Estrella et al., EMBO J. 2:987-992, 1983); streptomycin (Jones et al., Mol. Gen. Genet. 210:86-91, 1987); spectinomycin (Bretagne-Sagnard et al., Transgenic Res. 5:131-137, 1996); bleomycin (Hille et al., Plant Mol. Biol. 7:171-176, 1990); sulfonamide (Guerineau et al., Plant Mol. Biol. 15:127-136, 1990); bromoxynil (Stalker et al., Science 242:419-423, 1988); glyphosate (Shaw et al., Science 233:478-481, 1986); phosphinothricin (DeBlock et al., EMBO J. 6:2513-2518, 1987), and the like.

One option for use of a selective gene is a glufosinate-resistance encoding DNA and in one embodiment can be the phosphinothricin acetyl transferase (*pat*)*,* maize optimized *pat* gene or *bar* gene under the control of the Cassava Vein Mosaic Virus promoter. These genes confer resistance to bialaphos. See ,e.g., Wohlleben et al. (1988) Gene 70: 25-37); Gordon-Kamm et al., Plant Cell 2:603; 1990; Uchimiya et al., BioTechnology 11:835, 1993; White et al., Nucl. Acids Res. 18:1062, 1990; Spencer et al., Theor. Appl. Genet. 79:625-631, 1990; and Anzai et al., Mol. Gen. Gen. 219:492, 1989. A version of the *pat* gene is the maize optimized *pat* gene, described in U.S. Patent No. 6,096,947. Another selectable marker gene that can be used in the disclosed method is *dgt-28* which confers resistance glyphosate. In particular embodiments, *dgt-28* can be optimized for expression in dicotyledonous plant (see, e.g., US20130205440).

In addition, markers that facilitate identification of a plant cell containing the polynucleotide encoding the marker may be employed. Scorable or screenable markers are useful, where presence of the sequence produces a measurable product and can produce the product without destruction of the plant cell. Examples include a β-glucuronidase, or *uidA* gene (GUS), which encodes an enzyme for which various chromogenic substrates are known (for example, US Patents 5,268,463 and 5,599,670); chloramphenicol acetyl transferase (Jefferson et al. The EMBO Journal vol. 6 No. 13 pp. 3901-3907); and alkaline phosphatase. In a preferred embodiment, the marker used is beta-carotene or provitamin A (Ye et al, Science 287:303-305-(2000)). The gene has been used to enhance the nutrition of rice, but in this instance it is employed instead as a screenable marker, and the presence of the gene linked to a gene of interest is detected by the golden color provided. Unlike the situation where the gene is used for its nutritional contribution to the plant, a smaller amount of the protein suffices for marking purposes. Other screenable markers include the anthocyanin/flavonoid genes in general (See discussion at Taylor and Briggs, The Plant Cell (1990)2:115-127) including, for example, a R-locus gene, which encodes a product that regulates the production of anthocyanin pigments (red color) in plant tissues (Dellaporta et al., in Chromosome Structure and Function, Kluwer Academic Publishers, Appels and Gustafson eds., pp. 263-282 (1988)); the genes which control biosynthesis of flavonoid pigments, such as the maize C1 gene (Kao et al., Plant Cell (1996) 8: 1171-1179; Scheffler et al. Mol. Gen. Genet. (1994) 242:40-48) and maize C2 (Wienand et al., Mol. Gen. Genet. (1986) 203:202-207); the B gene (Chandler et al., Plant Cell (1989) 1:1175-1183), the p1 gene (Grotewold et al, Proc. Natl. Acad. Sci USA (1991) 88:4587-4591; Grotewold et al., Cell (1994) 76:543-553; Sidorenko et al., Plant Mol. Biol. (1999)39:11-19); the bronze locus genes (Ralston et al., Genetics (1988) 119:185-197; Nash et al., Plant Cell (1990) 2(11): 1039-1049), among others.

Further examples of suitable markers include the cyan fluorescent protein (CYP) gene (Bolte et al. (2004) J. Cell Science 117: 943-54 and Kato et al. (2002) Plant Physiol 129: 913-42), the yellow fluorescent protein gene (PHIYFP™ from Evrogen; see Bolte et al. (2004) J. Cell Science 117: 943-54); a lux gene, which encodes a luciferase, the presence of which may be detected using, for example, X-ray film, scintillation counting, fluorescent spectrophotometry, low-light video cameras, photon counting cameras or multiwell luminometry (Teeri et al. (1989) EMBO J. 8:343); a green fluorescent protein (GFP) gene (Sheen et al., Plant J. (1995) 8(5):777-84); and DsRed2 where plant cells transformed with the marker gene are red in color, and thus visually selectable (Dietrich et al. (2002) Biotechniques 2(2):286-293). Additional examples include a β-lactamase gene (Sutcliffe, Proc. Nat'l. Acad. Sci. U.S.A. (1978) 75:3737), which encodes an enzyme for which various chromogenic substrates are known (e.g., PADAC, a chromogenic cephalosporin); a xylE gene (Zukowsky et al., Proc. Nat'l. Acad. Sci. U.S.A. (1983) 80:1101), which encodes a catechol dioxygenase that can convert chromogenic catechols; an α-amylase gene (Ikuta et al., Biotech. (1990) 8:241); and a tyrosinase gene (Katz et al., J. Gen. Microbiol. (1983) 129:2703), which encodes an enzyme capable of oxidizing tyrosine to DOPA and dopaquinone, which in turn condenses to form the easily detectable compound melanin. Clearly, many such markers are available and known to one skilled in the art.

In certain embodiments, the nucleotide sequence can be optionally combined with another nucleotide sequence of interest. The term "nucleotide sequence of interest" refers to a nucleic acid molecule (which may also be referred to as a polynucleotide) which can be a transcribed RNA molecule as well as DNA molecule, that encodes for a desired polypeptide or protein, but also may refer to nucleic acid molecules that do not constitute an entire gene, and which do not necessarily encode a polypeptide or protein (e.g., a promoter). For example, in certain embodiments the nucleic acid molecule can be combined or "stacked" with another that provides additional resistance or tolerance to glyphosate or another herbicide, and/or provides resistance to select insects or diseases and/or nutritional enhancements, and/or improved agronomic characteristics, and/or proteins or other products useful in feed, food, industrial, pharmaceutical or other uses. The "stacking" of two or more nucleic acid sequences of interest within a plant genome can be accomplished, for example, via conventional plant breeding using two or more events, transformation of a plant with a construct which contains the sequences of interest, re-transformation of a transgenic plant, or addition of new traits through targeted integration via homologous recombination.

Such nucleotide sequences of interest include, but are not limited to, those examples provided below:

### 1. Genes or Sequence (e.g. iRNA) That Confer Resistance to Pests or Disease

(A) Plant Disease Resistance Genes. Plant defenses are often activated by specific interaction between the product of a disease resistance gene (R) in the plant and the product of a corresponding avirulence (Avr) gene in the pathogen. A plant variety can be transformed with cloned resistance gene to engineer plants that are resistant to specific pathogen strains. Examples of such genes include, the tomato Cf-9 gene for resistance to *Cladosporium fulvum* (Jones et al., 1994 Science 266:789), tomato *Pto* gene, which encodes a protein kinase, for resistance to *Pseudomonas syringae* pv. tomato (Martin et al., 1993 Science 262:1432), and *Arabidopsis* RSSP2 gene for resistance to *Pseudomonas syringae* (Mindrinos et al., 1994 Cell 78:1089).
(B) A *Bacillus thuringiensis* protein, a derivative thereof or a synthetic polypeptide modeled thereon, such as, a nucleotide sequence of a *Bt* δ-endotoxin gene (Geiser et al., 1986 Gene 48:109), and a vegetative insecticidal (VIP) gene (*see, e.g.,* Estruch et al. (1996) Proc. Natl. Acad. Sci. 93:5389-94). Moreover, DNA molecules encoding δ-endotoxin genes can be purchased from American Type Culture Collection (Rockville, Md.), under ATCC accession numbers 40098, 67136, 31995 and 31998.
(C) A lectin, such as, nucleotide sequences of several *Clivia miniata* mannose-binding lectin genes (Van Damme et al., 1994 Plant Molec. Biol. 24:825).
(D) A vitamin binding protein, such as avidin and avidin homologs which are useful as larvicides against insect pests. See U.S. Pat. No. 5,659,026.
(E) An enzyme inhibitor, e.g., a protease inhibitor or an amylase inhibitor. Examples of such genes include a rice cysteine proteinase inhibitor (Abe et al., 1987 J. Biol. Chem. 262:16793), a tobacco proteinase inhibitor I (Huub et al., 1993 Plant Molec. Biol. 21:985), and an α-amylase inhibitor (Sumitani et al., 1993 Biosci. Biotech. Biochem. 57:1243).
(F) An insect-specific hormone or pheromone such as an ecdysteroid and juvenile hormone a variant thereof, a mimetic based thereon, or an antagonist or agonist thereof, such as baculovirus expression of cloned juvenile hormone esterase, an inactivator of juvenile hormone (Hammock et al., 1990 Nature 344:458).
(G) An insect-specific peptide or neuropeptide which, upon expression, disrupts the physiology of the affected pest (J. Biol. Chem. 269:9). Examples of such genes include an insect diuretic hormone receptor (Regan, 1994), an allostatin identified in *Diploptera punctata* (Pratt, 1989), and insect-specific, paralytic neurotoxins (U.S. Pat. No. 5,266,361).
(H) An insect-specific venom produced in nature by a snake, a wasp, etc., such as a scorpion insectotoxic peptide (Pang, 1992 Gene 116:165).
(I) An enzyme responsible for a hyperaccumulation of monoterpene, a sesquiterpene, a steroid, hydroxamic acid, a phenylpropanoid derivative or another non-protein molecule with insecticidal activity.
(J) An enzyme involved in the modification, including the post-translational modification, of a biologically active molecule; for example, glycolytic enzyme, a proteolytic enzyme, a lipolytic enzyme, a nuclease, a cyclase, a transaminase, an esterase, a hydrolase, a phosphatase, a kinase, a phosphorylase, a polymerase, an elastase, a chitinase and a glucanase, whether natural or synthetic. Examples of such genes include, a callas gene (PCT published application WO93/02197), chitinase-encoding sequences (which can be obtained, for example, from the ATCC under accession numbers 3999637 and 67152), tobacco hookworm chitinase (Kramer et al., 1993 Insect Molec. Biol. 23:691), and parsley ubi4-2 polyubiquitin gene (Kawalleck et al., 1993 Plant Molec. Biol. 21:673).
(K) A molecule that stimulates signal transduction. Examples of such molecules include nucleotide sequences for mung bean calmodulin cDNA clones (Botella et al., 1994 Plant Molec. Biol. 24:757) and a nucleotide sequence of a maize calmodulin cDNA clone (Griess et al., 1994 Plant Physiol. 104:1467).
(L) A hydrophobic moment peptide. *See* U.S. Pat. Nos. 5,659,026 and 5,607,914; the latter teaches synthetic antimicrobial peptides that confer disease resistance.
(M) A membrane permease, a channel former or a channel blocker, such as a cecropin-β lytic peptide analog (Jaynes et al., 1993 Plant Sci. 89:43) which renders transgenic tobacco plants resistant to Pseudomonas solanacearum.
(N) A viral-invasive protein or a complex toxin derived therefrom. For example, the accumulation of viral coat proteins in transformed plant cells imparts resistance to viral infection and/or disease development effected by the virus from which the coat protein gene is derived, as well as by related viruses. Coat protein-mediated resistance has been conferred upon transformed plants against alfalfa mosaic virus, cucumber mosaic virus, tobacco streak virus, potato virus X, potato virus Y, tobacco etch virus, tobacco rattle virus and tobacco mosaic virus. See, for example, Beachy et al. (1990) Ann. Rev. Phytopathol. 28:451.
(O) An insect-specific antibody or an immunotoxin derived therefrom. Thus, an antibody targeted to a critical metabolic function in the insect gut would inactivate an affected enzyme, killing the insect. For example, Taylor et al. (1994) Abstract #497, Seventh Int'l. Symposium on Molecular Plant-Microbe Interactions shows enzymatic inactivation in transgenic tobacco via production of single-chain antibody fragments.
(P) A virus-specific antibody. See, for example, Tavladoraki et al. (1993) Nature 266:469, which shows that transgenic plants expressing recombinant antibody genes are protected from virus attack.
(Q) A developmental-arrestive protein produced in nature by a pathogen or a parasite. Thus, fungal endo α-1,4-D polygalacturonases facilitate fungal colonization and plant nutrient release by solubilizing plant cell wall homo-α-1,4-D-galacturonase (Lamb et al., 1992) Bio/Technology 10:1436. The cloning and characterization of a gene which encodes a bean endopolygalacturonase-inhibiting protein is described by Toubart et al. (1992 Plant J. 2:367).
(R) A developmental-arrestive protein produced in nature by a plant, such as the barley ribosome-inactivating gene that provides an increased resistance to fungal disease (Longemann et al., 1992). Bio/Technology 10:3305.
(S) RNA interference, in which an RNA molecule is used to inhibit expression of a target gene. An RNA molecule in one example is partially or fully double stranded, which triggers a silencing response, resulting in cleavage of dsRNA into small interfering RNAs, which are then incorporated into a targeting complex that destroys homologous mRNAs. See, e.g., Fire et al., US Patent 6,506,559; Graham et al.6,573,099.

### 2. Genes That Confer Resistance to a Herbicide

(A) Genes encoding resistance or tolerance to a herbicide that inhibits the growing point or meristem, such as an imidazalinone, sulfonanilide or sulfonylurea herbicide. Exemplary genes in this category code for mutant acetolactate synthase (ALS) (Lee et al., 1988 EMBOJ. 7:1241) also known as acetohydroxyacid synthase (AHAS) enzyme (Miki et al., 1990 Theor. Appl. Genet. 80:449).
(B) One or more additional genes encoding resistance or tolerance to glyphosate imparted by mutant EPSP synthase and aroA genes, or through metabolic inactivation by genes such as GAT (glyphosate acetyltransferase) or GOX (glyphosate oxidase) and other phosphono compounds such as glufosinate (pat and bar genes; DSM-2), and aryloxyphenoxypropionic acids and cyclohexanediones (ACCase inhibitor encoding genes). See, for example, U.S. Pat. No. 4,940,835, which discloses the nucleotide sequence of a form of EPSP which can confer glyphosate resistance. A DNA molecule encoding a mutant aroA gene can be obtained under ATCC Accession Number 39256, and the nucleotide sequence of the mutant gene is disclosed in U.S. Pat. No. 4,769,061. European patent application No. 0 333 033 and U.S. Pat. No. 4,975,374 disclose nucleotide sequences of glutamine synthetase genes which confer resistance to herbicides such as L-phosphinothricin. The nucleotide sequence of a phosphinothricinacetyl-transferase gene is provided in European application No. 0 242 246. De Greef et al. (1989) Bio/Technology 7:61 describes the production of transgenic plants that express chimeric bar genes coding for phosphinothricin acetyl transferase activity. Exemplary of genes conferring resistance to aryloxyphenoxypropionic acids and cyclohexanediones, such as sethoxydim and haloxyfop, are the Accl-S1, Accl-S2 and Accl-S3 genes described by Marshall et al. (1992) Theor. Appl. Genet. 83:435.
(C) Genes encoding resistance or tolerance to a herbicide that inhibits photosynthesis, such as a triazine (psbA and gs+ genes) and a benzonitrile (nitrilase gene). Przibilla et al. (1991) Plant Cell 3:169 describe the use of plasmids encoding mutant psbA genes to transform Chlamydomonas. Nucleotide sequences for nitrilase genes are disclosed in U.S. Pat. No. 4,810,648, and DNA molecules containing these genes are available under ATCC accession numbers 53435, 67441 and 67442. Cloning and expression of DNA coding for a glutathione S-transferase is described by Hayes et al. (1992) Biochem. J. 285:173.
(D) Genes encoding resistance or tolerance to a herbicide that bind to hydroxyphenylpyruvate dioxygenases (HPPD), enzymes which catalyze the reaction in which para-hydroxyphenylpyruvate (HPP) is transformed into homogentisate. This includes herbicides such as isoxazoles (EP418175, EP470856, EP487352, EP527036, EP560482, EP682659, U.S. Pat. No. 5,424,276), in particular isoxaflutole, which is a selective herbicide for maize, diketonitriles (EP496630, EP496631), in particular 2-cyano-3-cyclopropyl-1-(2-SO2CH3-4-CF3 phenyl)propane-1,3-dione and 2-cyano-3-cyclopropyl-l-(2-SO2CH3-4-2,3Cl2phenyl)propane-1,3-dione, triketones (EP625505, EP625508, U.S. Pat. No. 5,506,195), in particular sulcotrione, and pyrazolinates. A gene that produces an overabundance of HPPD in plants can provide tolerance or resistance to such herbicides, including, for example, genes described in U.S. Patent Nos. 6,268,549 and 6,245,968 and U.S. Patent Application, Publication No. 20030066102.
(E) Genes encoding resistance or tolerance to phenoxy auxin herbicides, such as 2,4-dichlorophenoxyacetic acid (2,4-D) and which may also confer resistance or tolerance to aryloxyphenoxypropionate (AOPP) herbicides. Examples of such genes include the α-ketoglutarate-dependent dioxygenase enzyme (aad-1) gene, described in U.S. Patent No. 7,838,733.
(F) Genes encoding resistance or tolerance to phenoxy auxin herbicides, such as 2,4-dichlorophenoxyacetic acid (2,4-D) and which may also confer resistance or tolerance to pyridyloxy auxin herbicides, such as fluroxypyr or triclopyr. Examples of such genes include the α-ketoglutarate-dependent dioxygenase enzyme gene (aad-12), described in WO 2007/053482 A2.
(G) Genes encoding resistance or tolerance to dicamba (see, e.g., U.S. Patent Publication No. 20030135879).
(H) Genes providing resistance or tolerance to herbicides that inhibit protoporphyrinogen oxidase (PPO) (see U.S. Pat. No. 5,767,373).
(I) Genes providing resistance or tolerance to triazine herbicides (such as atrazine) and urea derivatives (such as diuron) herbicides which bind to core proteins of photosystem II reaction centers (PS II) (See Brussian et al. (1989) EMBO J. 1989, 8(4): 1237-1245.

### 3. Genes That Confer or Contribute to a Value-Added Trait

(A) Modified fatty acid metabolism, for example, by transforming maize or Brassica with an antisense gene or stearoyl-ACP desaturase to increase stearic acid content of the plant (Knultzon et al., 1992) Proc. Nat. Acad. Sci. USA 89:2624.
(B) Decreased phytate content
   (1) Introduction of a phytase-encoding gene, such as the Aspergillus niger phytase gene (Van Hartingsveldt et al., 1993 Gene 127:87), enhances breakdown of phytate, adding more free phosphate to the transformed plant.
   (2) A gene could be introduced that reduces phytate content. In maize, this, for example, could be accomplished by cloning and then reintroducing DNA associated with the single allele which is responsible for maize mutants characterized by low levels of phytic acid (Raboy et al., 1990 Maydica 35:383).
(C) Modified carbohydrate composition effected, for example, by transforming plants with a gene coding for an enzyme that alters the branching pattern of starch. Examples of such enzymes include, Streptococcus mucus fructosyltransferase gene (Shiroza et al., 1988) J. Bacteriol. 170:810, Bacillus subtilis levansucrase gene (Steinmetz et al., 1985 Mol. Gen. Genel. 200:220), Bacillus licheniformis α-amylase (Pen et al., 1992 Bio/Technology 10:292), tomato invertase genes (Elliot et al., 1993), barley amylase gene (Sogaard et al., 1993 J. Biol. Chem. 268:22480), and maize endosperm starch branching enzyme II (Fisher et al., 1993 Plant Physiol. 102:10450).

The sequence of interest can also be a nucleotide sequence introduced into a predetermined area of the plant genome through homologous recombination. Methods to stably integrate a polynucleotide sequence within a specific chromosomal site of a plant cell via homologous recombination have been described within the art. For instance, site specific integration as described in US Patent Application Publication No. 2009/0111188 A1 involves the use of recombinases or integrases to mediate the introduction of a donor polynucleotide sequence into a chromosomal target. In addition, International Patent Application No. WO 2008/021207 describes zinc finger mediated-homologous recombination to stably integrate one or more donor polynucleotide sequences within specific locations of the genome. The use of recombinases such as FLP/FRT as described in US Patent No. 6,720,475, or CRE/LOX as described in US Patent No. 5,658,772, can be utilized to stably integrate a polynucleotide sequence into a specific chromosomal site. Finally, the use of meganucleases for targeting donor polynucleotides into a specific chromosomal location was described in Puchta et al., PNAS USA 93 (1996) pp. 5055-5060).

Other various methods for site specific integration within plant cells are generally known and applicable (Kumar et al., Trends in Plant Sci. 6(4) (2001) pp. 155-159). Furthermore, site-specific recombination systems that have been identified in several prokaryotic and lower eukaryotic organisms may be applied for use in plants. Examples of such systems include, but are not limited too; the R/RS recombinase system from the pSR1 plasmid of the yeast Zygosaccharomyces rouxii (Araki et al. (1985) J. Mol. Biol. 182: 191-203), and the Gin/gix system of phage Mu (Maeser and Kahlmann (1991) Mol. Gen. Genet. 230: 170-176).

While certain example *Agrobacterium* strains are described herein, the functionality of the novel transformation methods discussed could be moved to other *Agrobacterium* strains with the same criteria. Examples of other strains that could be used with the novel transformation method described herein include, but are not limited to, *Agrobacterium tumefaciens* strain C58, *Agrobacterium tumefaciens* strain Chry5, *Agrobacterium* rhizogenes strains, *Agrobacterium tumefaciens* strain EHA101, *Agrobacterium tumefaciens* strain EHA105, *Agrobacterium tumefaciens* strain MOG101, and *Agrobacterium tumefaciens* strain T37. Modified versions of such strains are described with more particularity in International Patent Application No. WO 2012/106222 A2.

In embodiments of the novel transformation method disclosed, mature soybean seeds are sterilized prior to cutting with the cutting tool and infection with *Agrobacterium.* Seeds may be sterilized using chlorine gas, mercuric chloride, immersion in sodium hypochloride, immersion in sodium carbonate, or other suitable methods known in the art. In embodiments, the seeds are imbibed using sterile water or other suitable hypotonic solutions prior to cutting and infection with *Agrobacterium.* Imbibing the seeds for 6-24 hours softens the seeds, saturates the cotyledons, and improves later shoot induction. Longer periods of imbibing may also be used, for example, up to 48 hours.

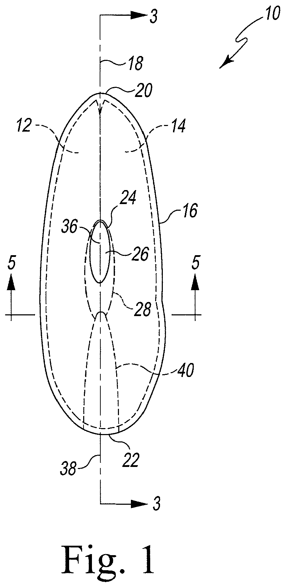

As described in greater detail below and illustrated in FIGS. 1-6, the soybean is prepared by splitting the cotyledons of the seeds along the hilum to separate the cotyledons, then removing the seed coat. Removal of a portion of the embryo axis leaves part of the axis attached to the cotyledons prior to transformation. In some examples, the removal of the embryo axis may be made by trimming of the embryo axis with a cutting tool or by breaking, tearing, and/or pinching the embryo axis during the splitting and separation of the cotyledons. Typically, between 1/3 and 1/2 of the embryo axis is left attached at the nodal end of the cotyledon.

Referring now to FIG. 1, a dicotyledonous soybean seed 10 is shown. The soybean seed 10 includes a pair of cotyledons 12, 14, which are encased in a seed coat 16. The soybean seed 10 has a longitudinal axis 18, which is defined along its maximum dimension, and extends through opposite longitudinal ends 20, 22 of the soybean seed 10. As shown in FIG. 1, the axis 18 extends between the cotyledons 12, 14.

The soybean seed 10 also includes a hilum 24 positioned between the ends 20, 22 of the soybean seed 10. In the illustrative embodiment, the hilum 24 includes an outer section 26 that is positioned outside of the seed coat 16 and an inner section 28 that is positioned under the seed coat 16.

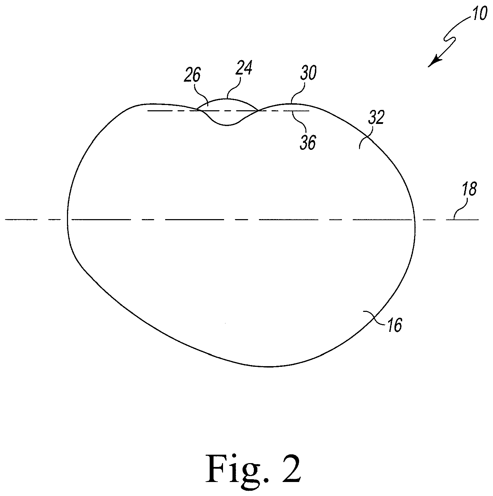

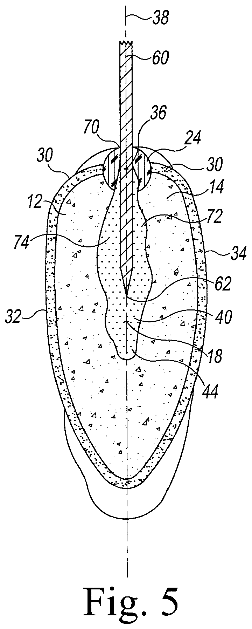

As shown in FIGS. 1-2, the hilum 24 is dorsally located above the cotyledons 12, 14. The outer section 26 of the hilum 24 is positioned on a dorsal side 30 of the soybean seed 10. The hilum 24 may also be viewed from the lateral side 32 (see FIG. 2) or the medial side 34 (see FIG. 5) of the soybean seed 10. As shown in FIG. 2, the hilum 24 has a longitudinal axis 36 that extends parallel to the overall longitudinal axis 18 of the seed 10. As shown in FIG. 1, the longitudinal axis 36 lies in a common plane 38 with the axis 18 of the seed 10.

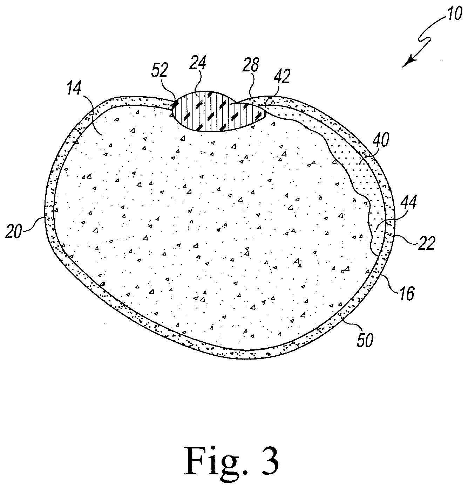

An embryonic axis 40 of the soybean seed 10 connects the cotyledon 12 to the cotyledon 14. The embryonic axis 40 is encased with the cotyledons 12, 14 in the seed coat 16. As shown in FIG. 1, the embryonic axis 40, like the hilum 24, is centered on the longitudinal axis 18 of the soybean seed 10. As shown in FIG. 3, the embryonic axis 40 extends from a tip 42 positioned above the inner section 28 of the hilum 24 to a base 44 positioned adjacent to the longitudinal end 20 of the seed 10. It should be appreciated that in other embodiments the embryonic axis 40 may not overlap with the hilum 24 such that the axis tip 42 is spaced apart from the inner section 28 of the hilum.

Referring to FIG. 3, the internal structure of the soybean seed 10 is shown in greater detail. The seed coat 16 includes a thin outer layer 50 that surrounds the cotyledons 12, 14 and the embryonic axis 40. The inner section 28 of the hilum 24 is attached to the underside of the layer 50, while the outer section 26 of the hilum 24 is connected to an edge 52 of the outer layer 50. The embryonic axis 40 extends around a portion of the outer circumference of the seed 10 from its tip 42 to its base 44 positioned adjacent to the seed end 20.

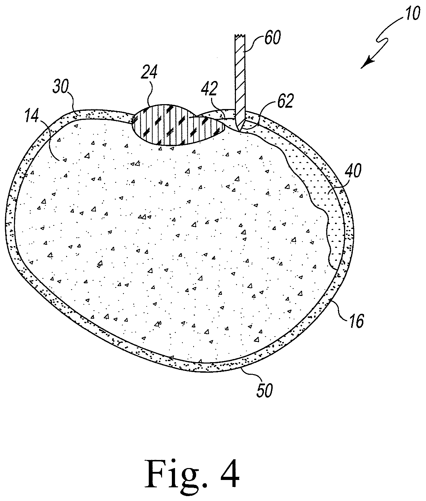

Referring to FIGS. 4-5, an illustrative technique for preparing the soybean seed 10 for transformation is shown. In the illustrative embodiment, a preparer grasps the seed 10 on its lateral side 32 and medial side 34 to orient the dorsal side 30 of the seed 10 upward. In other embodiments, the preparer may grasp only one of the sides 32, 34 and engage the opposite side against a vertical surface to hold the seed 10 in position. In still other embodiments the preparer may, for example, orient the dorsal side 30 so that it faces horizontally. In such embodiments, the preparer may grasp only one of the sides 32, 34 and engage the opposite side against a horizontal surface to hold the seed 10 in position.

A preparer may select a cutting tool 60 including a sharpened cutting edge 62 to cut the seed 10. As described above, the cutting tool 60 may take the form of a scalpel, razor blade, knife, and the like. In the illustrative embodiment, the preparer may orient the cutting tool 60 to trim the embryonic axis 40. To do so, the cutting tool 60 may be oriented with the cutting edge 62 pointing downward toward the seed 10, with the cutting edge 62 extends transverse to the axis 18 of the seed 10 (and hence the seed itself). As shown in FIG. 4, the tool 60 may be inserted into the seed 10, through the seed coat 16. The cutting edge 62 may be advanced through the embryonic axis 40 to separate the tip 42 of the axis 40 from the rest of the axis 40. As described above, typically, between 1/3 and 1/2 of the embryonic axis 40 may left attached. In other words, between 1/2 and 2/3 of the embryonic axis 40 may be trimmed along with the tip 42 from the rest of the embryonic axis 40.

In the illustrative embodiment, the cutting edge 62 of the tool 60 does not penetrate the cotyledons 12, 14 when the embryonic axis 40 is trimmed. In some embodiments, it may be desirable to wound the cotyledons 12, 14 by advancing the cutting edge 62 further into the seed 10. After trimming the embryonic axis 40, the preparer may withdraw the cutting tool 60 from the seed 10.

The preparer may also use the same cutting tool 60 (or a different cutting tool) to split the seed 10 into two cotyledon segments. To do so, the cutting tool 60 may be oriented with the cutting edge 62 pointing downward toward the seed 10. As shown in FIG. 5, the cutting edge 62 is aligned with plane 38 defined by the longitudinal axis 36 of the hilum 24 and the longitudinal axis 18 of the seed 10. To align the cutting tool 60, the preparer may orient the dorsal side 30 of the seed 10 upward and center the cutting edge 62 on the longitudinal axis 36 of the hilum 24. When the cutting tool 60 is properly aligned, the preparer may insert the cutting edge 62 into the seed 10, slicing through the seed coat 16 and the hilum 24 along the plane 38 and thereby creating an opening 70 in the seed 10. The preparer may continue to advance the cutting tool 60 into the seed 10, slicing the embryonic axis 40 into a medial section 72 attached to the cotyledon 12 and a lateral section 74 attached to the cotyledon 14. When the cutting edge 62 passes through the base 44 of the embryonic axis 40, the preparer may withdraw the tool 60 from the seed 10. In other embodiments, the preparer may continue to advance the tool 60 though the seed 10.

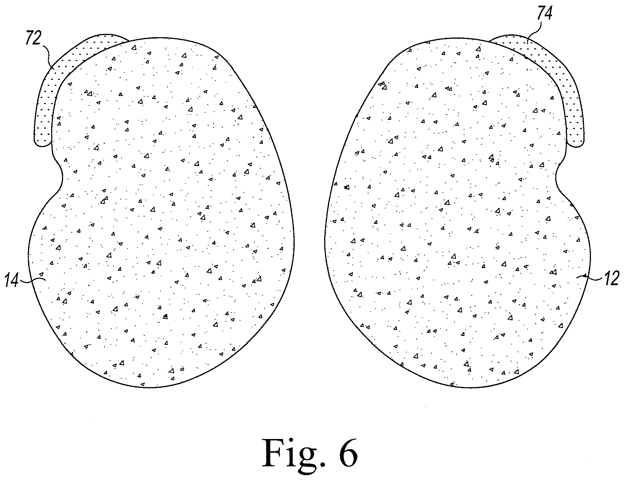

The preparer may separate the seed coat 16 from the cotyledons 12, 14 by widening the opening 70 to further expose the cotyledons 12, 14. The cotyledons 12, 14 may be removed from the seed coat 16, and the seed coat 16 discarded. As shown in FIG. 6, each cotyledon, which may be referred to as a split soybean seed or cotyledon segment, includes a section of the embryonic axis. In the illustrative embodiment, the cotyledon segment 12 includes the section 72 of the embryonic axis 40, while the cotyledon segment 14 includes the section 74 of the embryonic axis 40. Each of the cotyledon segments 12, 14 is then ready for further processing, including additional wounding or inoculation with an *Agrobacterium* culture.

Wounding of the split soybean seed is not required with the disclosed method, but is reported to increase transformation efficiency using other methods, including the cotyledonary node method and the meristem explant method. Wounding of the plant material may be facilitated by cutting, abrading, piercing, sonication, plasma wounding, or vacuum infiltration. Accordingly, additional wounding of the first cotyledon segment including a portion of the embryonic axis prior to transformation may be utilized as an embodiment of the present disclosure.

Split soybean seeds comprising a portion of an embryonic axis are typically inoculated with *Agrobacterium* culture containing a suitable genetic construct for about 0.5 to 3.0 hours, more typically for about 0.5 hours, followed by a period of co-cultivation on suitable medium for up to about 5 days. Explants which putatively contain a copy of the transgene arise from the culturing of the transformed split soybean seeds comprising a portion of an embryonic axis. These explants are identified and isolated for further tissue propagation.

Shoot induction may be facilitated by culturing explants in suitable induction media for a period of approximately two weeks, followed by culturing in media containing a selectable agent, such as glufosinate, for another two weeks. Alternating between media without a selectable agent, and media with a selectable agent, is preferred. Other protocols may be successfully employed wherein the shoot induction medium comprises a selectable agent or where the shoot induction medium and the selection medium are combined into a single medium that includes the selectable marker. Following a period of shoot induction, a tissue isolate containing a portion of the embryonic axis may be excised, and transferred to a suitable shoot elongation medium. In certain embodiments, the cotyledons may be removed, and a flush shoot pad excised containing the embryonic axis may be excised, by cutting at the base of the cotyledon. See Example 2.

Typically, one or more selective agents are applied to the split-seed explants following transformation. The selective agent kills or retards the growth of non-transformed soybean cells, and may help to eliminate the residual *Agrobacterium* cells. Suitable agents include glufosinate or Bialaphos. Other suitable agents include, but are not limited to, the herbicide glyphosate or the herbicide 2,4-D which act as both a selectable agent and shoot-inducing hormone. In addition, the selective agents can include various antibiotics, including spectinomycin, kanamycin, neomycin, paromomycin, gentamicin, and G418, depending on the selectable marker used. Depending on the agent used, selection for one to seven days may be appropriate.

Rooting of elongated shoots may be encouraged using suitable agents, including, but not limited to varying concentrations of auxins and cytokinins. For example the auxin, indole 3-butryic acid (IBA), may be incorporated into cell tissue culture medium that is used prior to transfer of the plant material to suitable rooting media known to those of skill in the art. Root formation takes approximately 1-4 weeks, more typically 1-2 weeks after exposure to IBA.

Cultivation of growing shoots may be accomplished by methods generally known in the art, leading to mature transgenic soybean plants. See, e.g., Example 2.

The presence of successful transgenic events may be confirmed using techniques known in the art, including, but not limited to Taqman™, PCR analysis, and Southern analysis of integrated selectable markers and/or reporter gene constructs in the soybean at any stage after infection and co-cultivation with *Agrobacterium;* phenotypic assay for plants or plant germplasm displaying evidence of a reporter construct; or selection of explants on suitable selection media.

In embodiments, the disclosed method may be used to facilitate breeding programs for the development of inbred soybean lines expressing genes of interest, and the development of elite soybean cultivars. Inbred soybean lines comprising stably-integrated transgenes may be crossed with other inbred soybean lines to produce hybrid plants expressing genes of interest. Introgression of a desired trait into elite soybean lines and hybrids may be rapidly achieved using the disclosed method and methods known in the art.

The following Examples are provided to illustrate particular features and/or aspects of the disclosure. These Examples should not be construed to limit the disclosure to the particular features or aspects described therein.

### EXAMPLES

### EXAMPLE 1: Vector Construction

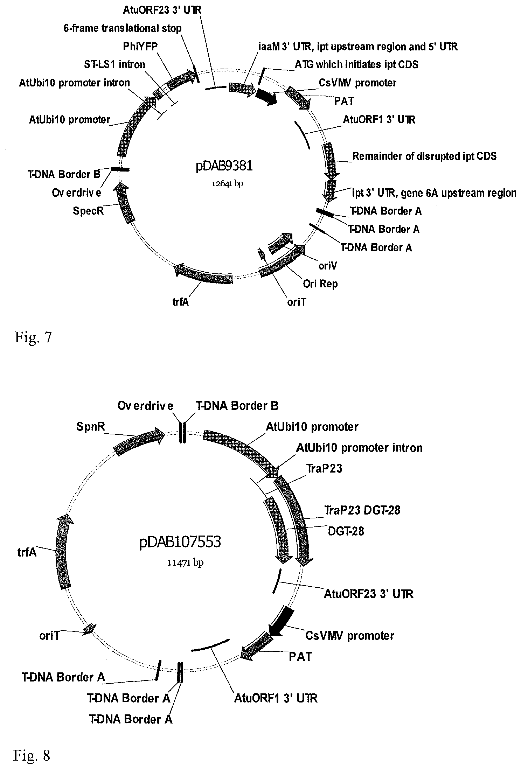

A single binary vector labeled as pDAB9381 (FIG. 7) was constructed using art-recognized procedures. pDAB9381 contains two Plant Transcription Units (PTUs). The first PTU (SEQ ID NO:1) consists of the Arabidopsis thaliana ubiquitin-10 promoter (AtUbi10 promoter; Callis et al., 1990) which drives the yellow fluorescence protein coding sequence (PhiYFP; Shagin et al., 2004) that contains an intron isolated from the Solanum tuberosum, light specific tissue inducible LS-1 gene (ST-LS1 intron; Genbank Acc No. X04753), and is terminated by the *Agrobacterium tumefaciens* open reading frame-23 3' untranslated region (AtuORF23 3'UTR; Gelvin et al., 1987). The second PTU (SEQ ID NO:2) was cloned within the isopentenyltransferase coding sequence (ipt CDS; Genbank Acc No. X00639.1), consisting of the Cassava Vein Mosaic Virus promoter (CsVMV promoter; Verdaguer et al., 1996) which is used to drive the phosphinothricin acetyl transferase coding sequence (PAT; Wohlleben et al., 1988), terminated by the A. tumefaciens open reading frame-1 3' untranslated region (AtuORF1 3'UTR; Huang et al., 1990). The resulting binary vector contained a visual reporter gene and a antibiotic selectable marker gene and was subsequently used for the transformation of soybean.

The binary vector, pDAB9381, was mobilized into the *Agrobacterium tumefaciens* strains of EHA101 and EHA105 (Hood et al., 1986) using electroporation. Individual colonies were identified which grew up on YEP media containing the antibiotic spectinomycin. Single colonies were isolated and the presence of the pDAB9381 binary vector was confirmed via restriction enzyme digestion.

### EXAMPLE 2: Agrobacterium-mediated Transformation of Soybean using Split-seeds Comprising a Portion of an Embryonic Axis

Seed preparation. A novel *Agrobacterium-mediated* soybean transformation protocol was developed. Mature soybean (Glycine max) cv. Maverick seeds were sterilized overnight with chlorine gas for sixteen hours. Following sterilization with chlorine gas, the seeds were placed in an open container in a Laminar™ flow hood to dispel the chlorine gas. Next, the sterilized seeds were imbibed with sterile H2O for sixteen hours in the dark using a black box at 24° C.

Preparation of split-seed soybeans. The split soybean seed comprising a portion of an embryonic axis protocol required preparation of soybean seed material which was cut longitudinally, using a cutting tool (e.g., #10 blade affixed to a scalpel). The cutting tool was inserted into the seed coat of the soybean seed (FIG. 5) along the hilum of the seed to separate and remove the seed coat, and to split the seed into two cotyledon sections (FIG. 6). Careful attention was made to partially remove the embryonic axis, wherein about 1/2 - 1/3 of the embryo axis remained attached to the nodal end of the cotyledon (FIG. 4). The method differed from previously-described transformation methods that result in the near-complete removal of the embryo axis when splitting the mature seed into two cotyledon sections.

Inoculation. The split soybean seeds comprising a partial portion of the embryonic axis were then immersed for 30 minutes in a solution of *Agrobacterium tumefaciens* strain EHA 101 or EHA 105 containing the pDAB9381 binary plasmid. The *Agrobacterium tumefaciens* solution was diluted to a final concentration of λ=0.6 OD650 before immersing the cotyledons comprising the embryo axis.

Co-cultivation. Following inoculation, the split soybean seed were allowed to co-cultivate with the *Agrobacterium tumefaciens* strain for 5 days on co-cultivation medium (Wang, Kan. Agrobacterium Protocols. 2. 1. New Jersey: Humana Press, 2006. Print.) in a Petri dish covered with a piece of filter paper.

Shoot induction. After 5 days of co-cultivation, the split soybean seeds were washed in liquid Shoot Induction (SI) media consisting of B5 salts, B5 vitamins, 28 mg/L Ferrous, 38 mg/L Na2EDTA, 30 g/L sucrose, 0.6 g/L MES, 1.11 mg/L BAP, 100 mg/L Timentin™, 200 mg/L cefotaxime, and 50 mg/L vancomycin (pH 5.7). The split soybean seeds were then cultured on Shoot Induction I (SI I) medium consisting of B5 salts, B5 vitamins, 7 g/L Noble agar, 28 mg/L Ferrous, 38 mg/L Na2EDTA, 30 g/L sucrose, 0.6 g/L MES, 1.11 mg/L BAP, 50 mg/L Timentin™, 200 mg/L cefotaxime, 50 mg/L vancomycin (pH 5.7), with the flat side of the cotyledon facing up and the nodal end of the cotyledon imbedded into the medium. After 2 weeks of culture, the explants from the transformed split soybean seed were transferred to the Shoot Induction II (SI II) medium containing SI I medium supplemented with 6 mg/L glufosinate (Liberty®).

Shoot elongation. After 2 weeks of culture on SI II medium, the cotyledons were removed from the explants and a flush shoot pad containing the embryonic axis was excised by making a cut at the base of the cotyledon. The isolated shoot pad from the cotyledon was transferred to Shoot Elongation (SE) medium. The SE medium consisted of MS salts, 28 mg/L Ferrous, 38 mg/L Na2EDTA, 30 g/L sucrose and 0.6 g/L MES, 50 mg/L asparagine, 100 mg/L L-pyroglutamic acid, 0.1 mg/L IAA, 0.5 mg/L GA3, 1 mg/L zeatin riboside, 50 mg/L Timentin™, 200 mg/L cefotaxime, 50 mg/L vancomycin, 6 mg/L glufosinate, 7 g/L Noble agar, (pH 5.7). The cultures were transferred to fresh SE medium every 2 weeks. The cultures were grown in a Conviron™ growth chamber at 24° C with an 18 h photoperiod at a light intensity of 80-90 µmol/m2sec.

Rooting. Elongated shoots which developed from the cotyledon shoot pad were isolated by cutting the elongated shoot at the base of the cotyledon shoot pad, and dipping the elongated shoot in 1 mg/L IBA (Indole 3-butyric acid) for 1-3 minutes to promote rooting. Next, the elongated shoots were transferred to rooting medium (MS salts, B5 vitamins, 28 mg/L Ferrous, 38 mg/L Na2EDTA, 20 g/L sucrose and 0.59 g/L MES, 50 mg/L asparagine, 100 mg/L L-pyroglutamic acid 7 g/L Noble agar, pH 5.6) in phyta trays.

Cultivation. Following culture in a Conviron™ growth chamber at 24° C, 18 h photoperiod, for 1-2 weeks, the shoots which had developed roots were transferred to a soil mix in a covered sundae cup and placed in a Conviron™ growth chamber (models CMP4030 and CMP3244, Controlled Environments Limited, Winnipeg, Manitoba, Canada) under long day conditions (16 hours light/8 hours dark) at a light intensity of 120-150 µmol/m2sec under constant temperature (22° C) and humidity (40-50%) for acclimatization of plantlets. The rooted plantlets were acclimated in sundae cups for several weeks before they were transferred to the greenhouse for further acclimatization and establishment of robust transgenic soybean plants.

### EXAMPLE 3: Confirmation of Transgenic Events via Agrobacterium-mediated Transformation of Split Soybean Seed Comprising a Portion of the Embryonic Axis

Transgenic soybean events containing a T-strand insert comprised of the YFP and PAT PTUs were produced using the novel *Agrobacterium-mediated* transformation of split soybean seeds comprising a portion of the embryonic axis transformation method described in Example 2.

A total of six independent transformation experiments, consisting of three experiments each for the EHA101 and EHA105 strains, were completed using the novel method. The T0 soybean putative transgenic events which were grown in the greenhouse were further confirmed by Taqman™ and PCR analysis of the PAT and YFP PTUs, respectively. The results of the average transformation frequency are presented in Table 1 and Table 2. Overall, the novel *Agrobacterium*-mediated transformation of split soybean seeds comprising a portion of the embryonic axis transformation method resulted in unprecedented transformation efficiencies of 13.3% (range: 4.6% -20.5%) and 20.3% (range: 14.0-26.5%). These transformation efficiency results are considerably higher than soybean transformation efficiencies which have been reported for soybean transformation methods described in the art using the cotyledonary node transformation method of Zeng et al. (2004) Plant Cell Rep 22: 478-482, and the half-seed transformation method of Paz et al. (2006) Plant Cell Rep 25: 206-213.

The novel *Agrobacterium-mediated* transformation of split soybean seeds comprising a portion of the embryonic axis transformation method resulted in a ∼3.7 fold increase in transformation frequency as compared to the soybean cotyledonary node transformation method of Zeng et al. (2004). Previous literature reports have indicated that the soybean cotyledonary node transformation method of Zeng et al. (2004) reached transformation efficiency levels as high as 5.9%. However, the novel *Agrobacterium-mediated* transformation of split soybean seeds comprising a portion of the embryonic axis transformation method results are still unexpectedly more efficient than the soybean cotyledonary node transformation method transformation efficiencies previously reported within the literature.

Likewise, the novel *Agrobacterium-mediated* transformation of split soybean seeds comprising a portion of the embryonic axis transformation method resulted in a ∼14 fold increase in transformation frequency as compared to the half-seed transformation method of Paz et al. (2006). Previous literature reports have indicated that the half-seed transformation method of Paz et al. (2006) resulted in an overall transformation efficiency of 3.8%. However, the novel *Agrobacterium-mediated* transformation of split soybean seeds comprising a portion of the embryonic axis transformation method results are still unexpectedly more efficient than the soybean half-seed transformation method transformation efficiencies previously reported within the literature.

**Table 1: First experiment of transgenic events produced from split soybean seeds comprising a portion of the embryonic axis. The average transformation frequency is the result of all the transformation experiments which were completed using the EHA101 and EHA105 Agrobacterium strains.**

| **Transformation Protocol** | **Number of Explants Treated** | **Number of Plants Sent to Greenhouse** | **Number of plants Survived in Greenhouse & Analyzed** | **Number of Plants with YFP & PAT Genes** | **Average Transformation frequency (% ± Std Err)** |
|---|---|---|---|---|---|
| Split-seed with embryo axis | 1,974 | 420 | 278 | 262 | 13.3 ± 0.02 |

**Table 2: Second experiment of transgenic events produced from split soybean seeds comprising a portion of the embryonic axis.**

| **Transformation Protocol** | **Number of Explants Treated** | **Number of Plants Regenerated After Selection** | **Number of Plants with YFP & PAT Genes** | **Average Transformation Frequency based on YFP Expression (% ± Std Err)** |
|---|---|---|---|---|
| Split-seed with embryo axis | 1,027 | 238 | 209 | 20.3 ± 3.6 |

### EXAMPLE 4: Determination of Heritability of the Transgenic Events Produced via Agrobacterium-mediated Transformation of Split Soybean Seeds with a Portion of the Embryonic Axis

The T0 soybean plants which were confirmed to contain copies of the YFP and PAT transgenes were self fertilized, and the resulting T1 seed from a total of 247 transgenic events were further screened for heritability. The transgenic T1 soybean seed was planted and grown in a greenhouse. At the V1-V2 stage of development (approximately 10-14 days after planting with 1-2 trifoliates), the plants were sprayed with a 1.0 % v/v solution of glufosinate (Liberty® (410 g ae/haL; Bayer Crop Sciences LLC). In addition, molecular analysis for both the PAT and YFP plant transcription units using the Taqman™ and PCR assays were completed on the T1 soybean plants. A total of 116 events (47%) were confirmed to be transgenic at the T1 generation as determined from the glufosinate herbicide tolerance, and the presence of the pat and yfp transgenes (Table 3). The remainder of the soybean events did not transmit the pat and yfp transgenes from the T0 to the T1 generation. The failure of these T1 soybean plants to inherent the transgene is a result of the production of T0 soybean events which were comprised of pat and yfp transgenes transformed into non-germline soybean tissues. The integration of transgenes into non-germline soybean tissue is a common technical problem which has been commonly described for other soybean transformation methods known in the art and is not unique to the *Agrobacterium -* mediated transformation of split soybean seeds comprising a portion of the embryonic axis transformation method.

**Table 3: Heritability of transgenic soybean events produced with split soybean seeds comprising a portion of the embryonic axis.**

| | **Number of T₁ Events Screened** | **Number of Heritable Events** | **Number of Non-germline Transformed Events** | **Percent Heritability** |
|---|---|---|---|---|
| Split-seed with embryo axis | 247 | 116 | 131 | 47% |

### EXAMPLE 5: Agrobacterium-mediated Transformation of Soybean using Split-seed Explants Comprising a Portion of an Embryonic Axis and Glyphosate as Selection Agent

The above described soybean transformation protocol (see Example 2) was utilized for transformation and selection of a transgene expressing a glyphosate tolerant trait. The DGT-28 gene (International Pat. Pub. No. WO2013116700) was incorporated into a gene expression cassette as a selectable and herbicide tolerant trait and used for transformation of soybean split-seed explants comprising a portion of an embryonic axis.

Initially, a dose response study was completed. To test the effect of glyphosate on the explants, glyphosate was added to media used from the shoot initiation stage and onwards. Glyphosate was tested from the range of 0.025-0.2 mM. Incorporation of glyphosate in tissue culture media within this concentration range has been reported in soybean (Clemente et al, 2000; Hinchee et al, 1996; Martinell et al, 2006; Martinell et al, 1999; Martinell et al, 2002), tobacco (Singer et al, 1985), cotton (Zhao et al, 2006) and wheat (Hu et al, 2003). Five concentrations of glyphosate were tested for culturing of un-transformed soybean explants. The concentrations incorporated into the SI-2 media were 0.025 mM, 0.05 mM, 0.075 mM, 0.1 mM and 0.2 mM. The un-transformed soybean explants were maintained on the same rate of glyphosate selection for all the successive stages of subculture from SI-2 media to the completion of the tissue culturing protocol. The numbers of healthy explants were counted at each subculture stage. Dead explants were discarded and healthy explants were subcultured further onto appropriate media.

At the end of culturing on SI-2 media containing various glyphosate concentrations (e.g. about 2 weeks of time), explants showed yellowing of the cotyledons. The extent of yellowness was directly proportional to the dose of glyphosate applied to the media. Growth of the explants was also retarded on glyphosate containing media. Next, the cotyledons were removed and the explants were subcultured from SI-2 to SE media. At the end of another two weeks of selection on SE media, significant differences in the growth of the explants were observed when the explants were exposed to different levels of glyphosate. The retarded growth of the explants was proportionate to the increasing dose of glyphosate. Similarly the number of dead explants was also proportional to the increasing dose of glyphosate. The number of dead explants increased as the dose of glyphosate was increased. At the highest dose utilized in this study (e.g., 0.2 mM), only one explants survived at the end of SE-1. Comparatively, almost all the explants were alive on the control media that did not contain glyphosate. Although some explants were able to survive at all the levels of glyphosate tested by the end of SE-1, all of the explants were dead at the end of SE-2, which is after six weeks on selection **(Table 4).** These results indicate that even at the lowest dose of glyphosate tested in tissue culture media (e.g., 0.025 mM), none of the soybean explants were able to survive after 6 weeks of glyphosate selection. Subsequently, three different concentrations of glyphosate (e.g., 0.025 mM, 0.05 mM and 0.1 mM) were incorporated into the selection schemes for *Agrobacterium-mediated* transformation of the soybean split-seed explants comprising a portion of an embryonic axis.

**Table 4: Results of dose response study. The table provides the number of explants that were advanced through each stage of subculture on different media types containing a range of glyphosate.**

| **Treatmen t Number** | **Glyphosate Concentratio n** | **Number of Explants at Various Stages of Selection** | | | | | |
|---|---|---|---|---|---|---|---|
| | | CC | SI-1 | SI-2 | SE-1 | SE-2 | End of SE-2 |
| 1 | 0 mM | 49 | 47 | 46 | 45 | 45 | 45 |
| 2 | 0.025 mM | 49 | 44 | 43 | 41 | 38 | 0 |
| 3 | 0.05 mM | 49 | 46 | 45 | 45 | 38 | 0 |
| 4 | 0.075 mM | 49 | 48 | 47 | 45 | 21 | 0 |
| 5 | 0.1 mM | 49 | 45 | 44 | 41 | 13 | 0 |
| 6 | 0.2 mM | 49 | 43 | 42 | 31 | 1 | 0 |

A single binary vector labeled as pDAB107553 (FIG. 8) was constructed using art-recognized procedures. pDAB107553 contains two Plant Transcription Units (PTUs). The first PTU (SEQ ID NO:3) consists of the Arabidopsis thaliana ubiquitin-10 promoter (AtUbi10 promoter; Callis et al., 1990) which drives the dgt-28 coding sequence (DGT-28; Shagin International Pat. Pub. No. WO2013116700) that contains the chlorophyll transit peptide, TraP23 (Trap23; International Pat. Pub. No. WO2013116764), and is terminated by the *Agrobacterium tumefaciens* open reading frame-23 3' untranslated region (AtuORF23 3'UTR; Gelvin et al., 1987). The second PTU (SEQ ID NO:4) consists of the Cassava Vein Mosaic Virus promoter (CsVMV promoter; Verdaguer et al., 1996) which is used to drive the phosphinothricin acetyl transferase coding sequence (PAT; Wohlleben et al., 1988), terminated by the A. tumefaciens open reading frame-1 3' untranslated region (AtuORF1 3'UTR; Huang et al., 1990). The resulting binary vector contained a herbicide tolerant selectable marker/resistance gene and an antibiotic selectable marker gene and was subsequently used for the transformation of soybean.

The binary vector, pDAB107553, was mobilized into the *Agrobacterium tumefaciens* strain of EHA105 (Hood et al., 1986) using electroporation. Individual colonies were identified which grew up on YEP media containing the antibiotic spectinomycin. Single colonies were isolated and the presence of the pDAB107553 binary vector was confirmed via restriction enzyme digestion.

The novel *Agrobacterium-mediated* soybean split-seed comprising a portion of an embryonic axis transformation method described in Example 2 was used to transform mature soybean (Glycine max) cv. Maverick seeds with an *Agrobacterium* strain harboring pDAB107553. Three different concentrations of glyphosate (e.g., 0.025 mM, 0.05 mM and 0.1 mM) were incorporated into the selection schemes for the soybean split-seed comprising a portion of an embryonic axis transformation method.

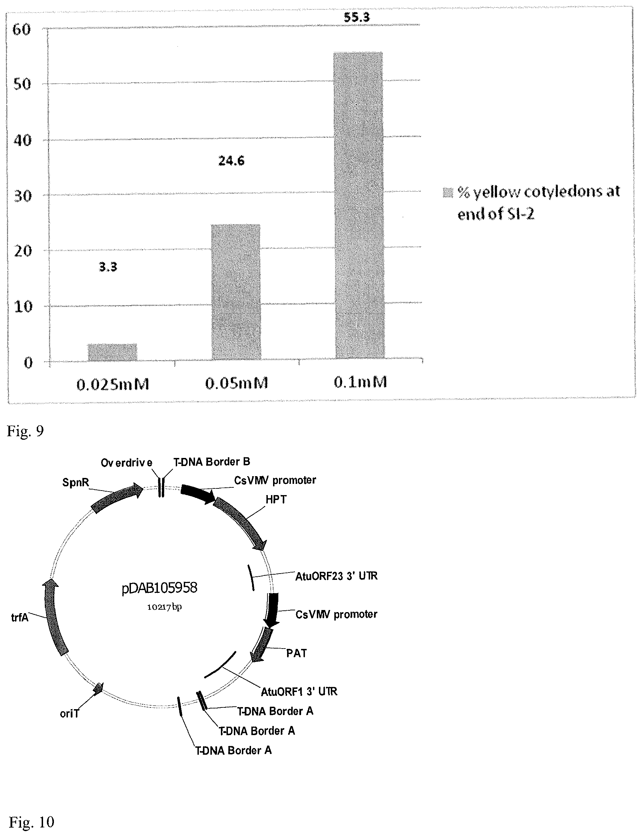

As the transformation experiment progressed through various subculture stages, the health of the soybean explants deteriorated. The greatest adverse physiological effects were observed at the highest level of glyphosate (e.g., 0.1 mM) and the adverse physiological effects reduced with decreasing concentrations of glyphosate. After the first selection cycle (i.e., two weeks on SI-2 media), yellowing of the cotyledons was noted for soybean explants culturing on glyphosate selection. The intensity of cotyledon yellowing was more prominent in soybean explants cultured on greater rates of glyphosate (FIG. 9).

While the soybean explants were being maintained on media containing glyphosate, it was observed that the explants were showing softening on the surfaces of the plant tissues that were directly in contact with the media. The surface of the explants was turning soft and creamy in consistency. The softening effect was very pronounced at the end of SE-1 stage onwards. Accordingly, the explants were transferred to media that did not contain the glyphosate selective agent. Half of the replication 1 and replication 2 explants were transferred onto media that did not contain any glyphosate selection agent at the end of the SE-1 stage. Subsequently, all of the explants were moved to media that did not contain any glyphosate selection at the end of SE-2 (Table 5). Since the softening was very pronounced in replication 1 and replication 2, half of the replication 3 explants were transferred onto media that did not contain any glyphosate selection agent at the end of SI-2 stage. By the end of stage SE-1, all of the replication 3 explants were transferred onto media that did not contain any glyphosate selection agent.

**Table 5: Shows the number of explants advanced through each stage of subculture. The numbers of explants at each stage of culturing on selection are provided, while the underlined numbers shows the number of explants moved to no selection media.**

| **Selection scheme 1 SI-1: 0 mM; SI-2: 0.025 mM/0.00 mM: SE-1: 0.025 mM/0.00 mM** | | | | | | | | |
|---|---|---|---|---|---|---|---|---|
| Replication | Number of explants surviving at various stages of selection | | | | | | | |
| | CC | SI-1 | SI-2 | SE-1 | SE-2 | SE-3 | SE-4 | SE-5 |
| R1 | 141 | 124 | 123 | 123/- | 65/57 | 121 | 121 | 30 |
| R2 | 141 | 126 | 118 | 118/- | 58/58 | 106 | 104 | --- |
| R3 | 113 | 96 | 93 | 48/45 | 93 | 85 | --- | --- |

| **Selection scheme 2 SI-1: 0 mM; SI-2: 0.05 mM/0.00 mM: SE-1: 0.025 mM/0.00 mM** | | | | | | | | |
|---|---|---|---|---|---|---|---|---|
| Replication | Number of explants surviving at various stages of selection | | | | | | | |
| | CC | SI-1 | SI-2 | SE-1 | SE-2 | SE-3 | SE-4 | SE-5 |
| R1 | 141 | 124 | 122 | 120/- | 64/55 | 118 | 86 | 20 |
| R2 | 141 | 127 | 119 | 117/- | 59/54 | 93 | 91 | --- |
| R3 | 114 | 98 | 93 | 48/45 | 93 | 78 | --- | --- |

| **Selection scheme 3 SI-1: 0 mM; SI-2: 0.1 mM/0.00 mM: SE-1: 0.025 mM/0.00 mM** | | | | | | | | |
|---|---|---|---|---|---|---|---|---|
| Replication | Number of explants surviving at various stages of selection | | | | | | | |
| | CC | SI-1 | SI-2 | SE-1 | SE-2 | SE-3 | SE-4 | SE-5 |
| R1 | 141 | 124 | 123 | 119/- | 62/48 | 108 | 102 | 16 |
| R2 | 141 | 126 | 118 | 116/- | 55/54 | 88 | 85 | --- |
| R3 | 113 | 96 | 93 | 43/47 | 90 | 70 | --- | --- |

For all three replications, the softening effect of the explants did not end when the explants were transferred to media that did not contain glyphosate, and the explants softening continued in all the subsequent stages of the subculture. These results indicate that once the explants have been exposed to glyphosate, the exposure initiates the softening of the tissue which persists even when explants are later moved to glyphosate free media.

Samples from all the surviving shoots were isolated from the explants and were sent for molecular analysis to confirm if they were transgenic. After confirming the presence or absence of the transgene in the shoots, the transgenic soybean events were moved to rooting media and were subsequently sent to the greenhouse for acclimatization and for further growth in the soil.

As shown in Table 6, each particular treatment of glyphosate within the tissue culture media resulted in the production of transgenic shoots. Despite the presence of a softening effect on the explants exposed to glyphosate, transgenic shoots were produced for all of the tested concentrations of glyphosate. Furthermore, the shoots were moved to rooting media and many of the shoots rooted successfully. Overall, the soybean split-seed comprising a portion of an embryonic axis transformation resulted in a transformation efficiency of 5.3% to 3.9%. However, there was some loss of transgenic plants, which did not produce roots. In addition, the transformation frequency was reduced when transgenic plants did not survive in the greenhouse. As such, a 0.63% transformation frequency (e.g., resulting in 8 transgenic plants) was obtained for rooted transgenic plants which survived in the greenhouse and were able to set seed. From these experiments, transgenic soybean plants were produced using the soybean split-seed comprising a portion of an embryonic axis transformation method that incorporated glyphosate as the selection agent.

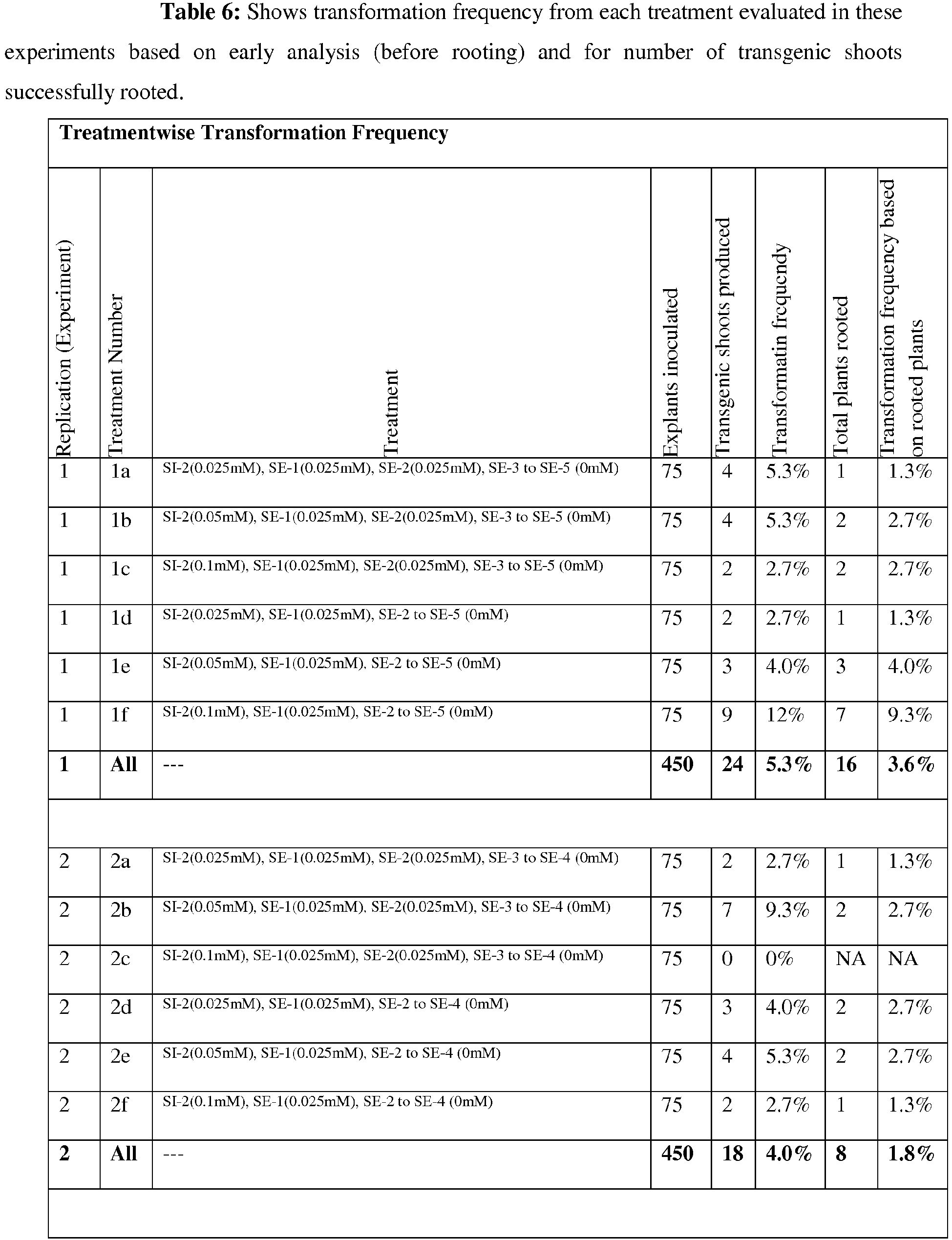

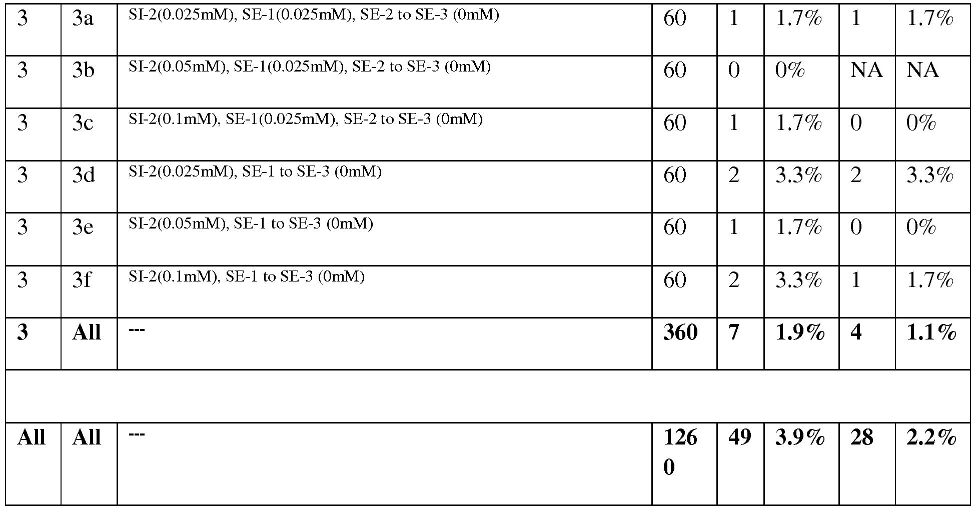

### EXAMPLE 6: Agrobacterium-mediated Transformation of Soybean using Split-seed Explants Comprising a Portion of an Embryonic Axis and Hygromycin as Selection Agent

The above described soybean transformation protocol (see, Example 2) was utilized for transformation and selection of a transgene conferring hygromycin tolerance. The *hpt* gene (Gritz, L. and Davies, J. (1983) Gene 25 (2-3); 179-188) was incorporated into a gene expression cassette as a selectable marker and used for soybean split-seed comprising a portion of an embryonic axis transformation.

Initially, a dose response study was completed. To test the effect of hygromycin on the explants, hygromycin was added to media used from the shoot initiation stage and onwards. Hygromycin was tested from the range of 5 mg/L to 25 mg/L on *Glycine max* c.v. Jack and *Glycine max* c.v. Maverick plants. Five concentrations of hygromycin were obtained from two different providers, Sigma Aldrich (St. Louis, MO) and Phytotech (Shawnee Mission, KS), and tested for culturing of un-transformed soybean explants. The concentrations incorporated into the SI-2 media were 5 mg/L, 10 mg/L, 15 mg/L, 20 mg/L and 25 mg/L. The un-transformed soybean explants were maintained on the same rate of hygromycin selection for all the successive stages of subculture from SI-2 media to the completion of the tissue culturing protocol. The numbers of healthy explants were counted at each subculture stage. Dead explants were discarded and healthy explants were subculture further onto appropriate media.

At the end of culturing on the media containing hygromycin, significant differences in the growth of the explants were observed when the explants were exposed to different levels of hygromycin **(Table 7).** The retarded growth of the explants was proportionate to the increasing dose of hygromycin. Similarly, the number of dead explants was also proportional to the increasing dose of hygromycin. The number of dead explants increased as the dose of hygromycin was increased. At the higher doses utilized in this study (10 mg/L to 25 mg/L) only a small number of explants survived. Likewise, a large number of explants were able to survive on media containing 5 mg/L of hygromycin. Comparatively, almost all the explants were alive on the control media that did not contain hygromycin. Although a large number of the explants were able to survive at all the levels of hygromycin tested by the end of SE-1, most of the explants were dead at the end of SE-2 for explants cultured on media containing hygromycin at concentrations of 10 mg/L to 25 mg/L. These results indicate that a dose of 5 mg/L of hygromycin tested is acceptable for use in tissue culture media.

**Table 7: Results of dose response study. The table provides the number of explants that were advanced through each stage of subculturing on different media types containing a range of hygromycin.**

| **Conditions** | **Rep. No.** | **Hygromycin** | **CC** | **SI-1** | **SI-2** | **SE-1** | **SE-2** | **End of SE-2** |
|---|---|---|---|---|---|---|---|---|
| *Gly. max* c.v.Maverick With Hygromycin from Sigma Aldrich (St. Louis, MO) | 1 | 0 mg/L | 45 | 44 | 41 | 41 | 38 | 38 |
| | 2 | 5 mg/L | 45 | 41 | 37 | 36 | 36 | 23 |
| | 3 | 10 mg/L | 45 | 42 | 38 | 38 | 37 | 4 |
| | 4 | 15 mg/L | 45 | 42 | 40 | 40 | 28 | 2 |
| | 5 | 20 mg/L | 45 | 45 | 44 | 43 | 26 | 3 |
| | 6 | 25 mg/L | 45 | 42 | 37 | 37 | 19 | 3 |
| *Gly. max* c.v.Maverick With Hygromycin from Phytotech (Shawnee Mission, KS) | 1 | 0 mg/L | 50 | 48 | 48 | 46 | 46 | 46 |
| | 2 | 5 mg/L | 50 | 48 | 47 | 47 | 47 | 5 |
| | 3 | 10 mg/L | 50 | 48 | 46 | 44 | 40 | 0 |
| | 4 | 15 mg/L | 50 | 49 | 47 | 47 | 18 | 0 |
| | 5 | 20 mg/L | 50 | 49 | 48 | 46 | 13 | 0 |
| | 6 | 25 mg/L | 50 | 49 | 48 | 47 | 2 | 0 |
| *Gly. max* c.v.Jack With Hygromycin from Sigma Aldrich (St. Louis, MO) | 1 | 0 mg/L | 50 | 48 | 30 | 29 | 29 | 29 |
| | 2 | 5 mg/L | 50 | 48 | 31 | 31 | 28 | 0 |
| | 3 | 10 mg/L | 50 | 49 | 31 | 30 | 26 | 0 |
| | 4 | 15 mg/L | 50 | 49 | 31 | 29 | 6 | 0 |
| | 5 | 20 mg/L | 50 | 49 | 31 | 30 | 4 | 0 |
| | 6 | 25 mg/L | 50 | 49 | 31 | 27 | 2 | 0 |
| *Gly. max* c.v. Jack With Hygromycin from Phytotech (Shawnee Mission, KS) | 1 | 0 mg/L | 50 | 47 | 29 | 28 | 28 | 28 |
| | 2 | 5 mg/L | 50 | 44 | 29 | 28 | 17 | 0 |
| | 3 | 10 mg/L | 50 | 50 | 29 | 28 | 11 | 0 |
| | 4 | 15 mg/L | 50 | 48 | 29 | 28 | 8 | 0 |
| | 5 | 20 mg/L | 50 | 48 | 29 | 27 | 3 | 0 |
| | 6 | 25 mg/L | 50 | 48 | 30 | 28 | 5 | 0 |

A single binary vector labeled as pDAB105958 (FIG. 10) was constructed using art-recognized procedures. pDAB105958 contains two Plant Transcription Units (PTUs). The first PTU (SEQ ID NO:5) consists of the Cassava Vein Mosaic Virus promoter (CsVMV promoter; Verdaguer et al., 1996) which drives the hpt coding sequence (HPT; Gritz and Davies, 1983), and is terminated by the *Agrobacterium tumefaciens* open reading frame-23 3' untranslated region (AtuORF23 3'UTR; Gelvin et al., 1987). The second PTU (SEQ ID NO:6) consists of the Arabidopsis thaliana ubiquitin-10 promoter (AtUbi10 promoter; Callis et al., 1990) which drives the phosphinothricin acetyl transferase coding sequence (PAT; Wohlleben et al., 1988), terminated by the A. tumefaciens open reading frame-1 3' untranslated region (AtuORF1 3'UTR; Huang et al., 1990). The resulting binary vector contained a herbicide tolerant selectable marker/resistance gene and an antibiotic selectable marker gene and was subsequently used for the transformation of soybean.

The binary vector, pDAB105958, was mobilized into the *Agrobacterium tumefaciens* strain of EHA105 (Hood et al., 1986) using electroporation. Individual colonies were identified which grew up on YEP media containing the antibiotic spectinomycin. Single colonies were isolated and the presence of the pDAB105958 binary vector was confirmed via restriction enzyme digestion.

The novel *Agrobacterium-mediated* soybean split-seed comprising a portion of an embryonic axis transformation method described in Example 2 was used to transform mature soybean (Glycine max) cv. Maverick seeds with an *Agrobacterium* strain harboring pDAB105958. Three different concentrations of hygromycin (e.g., 8 mg/L, 5 mg/L, and 3 mg/L) were incorporated into the selection schemes for the soybean split-seed comprising a portion of an embryonic axis transformation method.

While the soybean explants were being maintained on media containing hygromycin, it was observed that the explants were showing softening on the surfaces of the plant tissues that were directly in contact with the media. Accordingly, the explants were transferred to media that did not contain the hygromycin selective agent at the SE-3 stage of culturing (Table 8).

**Table 8: Shows the concentration of hygromycin and the number of explants surviving at end of each stage of selection.**

| **Experiment Number** | **CC** | **SI-1** | **SI-2** | **SE-1** | **SE-2** | **SE-3** | **SE-4** | **SE-5** |
|---|---|---|---|---|---|---|---|---|
| HygDR-5 | 200 | 192 | 187 (8mg/L) | 179 (5mg/L) | 177 (3mg/L) | 157 (0mg/L) | 34 (0mg/L) | 24 (0mg/L) |
| HygDR-5a | 200 | 193 | 189 (5mg/L) | 186 (5mg/L) | 186 (3mg/L) | 151 (0mg/L) | 56 (0mg/L) | 23 (0mg/L) |
| HygDR-6 | 200 | 185 | 185 (8mg/L) | 184 (5mg/L) | 184 (5mg/L) | 95 (0mg/L) | 11 (0mg/L) | 2 (0mg/L) |
| HygDR-6a | 200 | 189 | 189 (5mg/L) | 181 (5mg/L) | 181 (5mg/L) | 166 (0mg/L) | 48 (0mg/L) | 2 (0mg/L) |
| HygDR-7 | 200 | 187 | 187 (8mg/L) | 187 (5mg/L) | 186 (5mg/L) | 108 (0mg/L) | 22 (0mg/L) | 12 (0mg/L) |
| HygDR-7a | 200 | 187 | 181 (5mg/L) | 181 (5mg/L) | 180 (5mg/L) | 162 (0mg/L) | 28 (0mg/L) | 28 (0mg/L) |

Samples from all the surviving shoots were isolated from the explants and were sent for molecular analysis to confirm if they were transgenic. After confirming the presence or absence of the transgene in the shoots, the transgenic soybean events were moved to rooting media and were subsequently sent to the greenhouse for acclimatization and for further growth in the soil. As shown in Table 9, half of the treatments of hygromycin within the tissue culture media resulted in the production of transgenic shoots. Despite the presence of a softening effect on the explants exposed to hygromycin, transgenic shoots were produced on media containing the hygromycin selective agent. From these experiments, transgenic soybean shoots were produced using the soybean split-seed comprising a portion of an embryonic axis transformation method that incorporated hygromycin as the selection agent.

**Table 9: Shows transformation frequency from each treatment evaluated in these experiments based on early analysis (before rooting) and for number of transgenic shoots successfully produced.**

| **Experiment Number** | **Treatment Applied at Various Stages** | **Number of Explants** | **Transgenic Shoots Produced** | **% Shoots Produced** |
|---|---|---|---|---|
| HygDR-5a | SI-2 (**5mg/L**), SE-1 (**5mg/L**), SE-2 (**3mg/L**), SE-3 (0mg/L), SE-4 (0mg/L), SE-5 (0mg/L) | 200 | 0 | 0% |
| HygDR-5 | SI-2 **(8mg/L),** SE-1 **(5mg/L),** SE-2 **(3mg/L),** SE-3 (0mg/L), SE-4 (0mg/L), SE-5 (0mg/L) | 200 | 6 | 3% |
| | | | | |
| HygDR-6a | SI-2 (**5mg/L**), SE-1 (**5mg/L**), SE-2 (**5mg/L**), SE-3 (0mg/L), SE-4 (0mg/L), SE-5 (0mg/L) | 200 | 0 | 0% |
| HygDR-6 | SI-2 (**8mg/L**), SE-1 (**5mg/L**), SE-2 (**5mg/L**), SE-3 (0mg/L), SE-4 (0mg/L), SE-5 (0mg/L) | 200 | 0 | 0% |
| | | | | |
| HygDR-7a | SI-2 (**5mg/L**), SE-1 (**5mg/L**), SE-2 (**5mg/L**), SE-3 (0mg/L), SE-4 (0mg/L), SE-5 (0mg/L) | 200 | 2 | 1% |
| HygDR-7 | SI-2 (**8mg/L**), SE-1 (**5mg/**L), SE-2 (**5mg/L**), SE-3 (0mg/L), SE-4 (0mg/L), SE-5 (0mg/L) | 200 | 3 | 1.50% |
| **Total** | | **1200** | 11 | **0.92%** |

## Claims

1. A method of producing a transgenic event, the method comprising:
inserting a cutting tool into a seed coat of a soybean seed to form a first cotyledon segment and a second cotyledon segment, wherein the seed includes an embryonic axis;
inoculating the first cotyledon segment including a 1/4, 1/3, 1/2, 2/3, 3/4 or the full length portion of the embryonic axis with *Agrobacterium,* the *Agrobacterium* including at least one transgene; and
culturing the inoculated cotyledon segment to produce a transgenic event in a transformed plant cell.

2. The method of claim 1, further comprising at least one of the following:
(i) removing the seed coat from the first cotyledon segment and the second cotyledon segment prior to inoculating the first cotyledon segment,
(ii) inserting the cutting tool comprises cutting the embryonic axis longitudinally to retain a portion of the embryonic axis with each cotyledon segment,
(iii) inserting the cutting tool comprises cutting the soybean seed longitudinally along a hilum of the soybean seed,
(iv) inserting the cutting tool comprises advancing the cutting tool into the embryonic axis of the soybean seed prior to forming the first and second cotyledon segments, or
(v) the transgene is selected from the group consisting of insecticidal resistance gene, herbicide tolerance gene, nitrogen use efficiency gene, water use efficiency gene, nutritional quality gene, DNA binding gene, and selectable marker gene, wherein preferably the transgene comprises a *pat, dgt-28,* or *hpt* selectable marker gene.

3. The method of claim 2 (ii), wherein the embryonic axis of the soybean seed has an uncut length prior to inserting the cutting tool, and the portion of the embryonic axis of the first cotyledon segment has a length that is
(a) about 1/3 to 1/2 of the uncut length of the embryonic axis of the soybean seed, or
(b) equal to the uncut length.

4. The method of claim 3 (a), further comprising cutting the portion of the embryonic axis of the first cotyledon segment to the length that is about 1/3 to 1/2 of the uncut length of the embryonic axis of the soybean seed.

5. The method of claim 2 (iii), further comprising locating the hilum on an outer surface of the soybean seed, and orienting the cutting tool relative to the soybean seed to align the cutting tool with the hilum prior to inserting the cutting tool, preferably wherein each cotyledon segment includes a portion of the hilum.

6. The method of claim 2 (iii), wherein the soybean seed has a longitudinal axis extending through the hilum, the longitudinal axis dividing the soybean seed into a first side and a second side, the method further comprising grasping the first side of the soybean seed prior to inserting the cutting tool.

7. The method of claim 1, wherein the method further comprises producing a whole, fertile, soybean plant from the cultured inoculated cotyledon segment, wherein the whole, fertile, soybean plant comprises the transgenic event.

8. A cultured inoculated cotyledon segment produced by the method of claim 1, wherein the cotyledon segment comprises the transgenic event.

9. A method of producing a transgenic event, the method comprising:
inserting a cutting tool into a seed coat of a soybean seed to form a cotyledon segment including a 1/4, 1/3, 1/2, 2/3, 3/4 or the full length portion of the embryonic axis,
inoculating the cotyledon segment including the portion of the embryonic axis with *Agrobacterium,* the *Agrobacterium* including at least one transgene,
culturing the inoculated cotyledon segment to produce a transgenic event in a transformed plant cell, and
producing a whole, fertile plant from the cultured inoculated cotyledon segment, the whole, fertile plant including the transgenic event,
preferably wherein the seed is a dicotyledonous seed.

10. The method of claim 9, wherein inserting the cutting tool into the seed coat of the seed forms a first cotyledon segment and a second cotyledon segment, each cotyledon segment including a portion of the embryonic axis.

## Patentansprüche

1. Ein Verfahren zum Herstellen eines transgenen Ereignisses, wobei das Verfahren umfasst:
Einführen eines Schneidwerkzeugs in eine Samenhülle eines Sojabohnensamens, um ein erstes Kotyledonensegment und ein zweites Kotyledonensegment zu bilden, wobei der Samen eine embryonale Achse umfasst;
Beimpfen des ersten Kotyledonensegments, einschließlich eines 1/4-, 1/3-, 1/2-, 2/3-, 3/4- oder den vollständigen Anteil der embryonalen Achse mit *Agrobakterium,* wobei das *Agrobakterium* wenigstens ein Transgen enthält; und
Kultivieren des beimpften Kotyledonensegments, um ein transgenes Ereignis in einer transformierten Pflanzenzelle herzustellen.

2. Das Verfahren gemäß Anspruch 1, außerdem umfassend wenigstens eines der Folgenden:
(i) Entfernen der Samenhülle von dem ersten Kotyledonensegment und dem zweiten Kotyledonensegment vor dem Beimpfen des ersten Kotyledonensegments,
(ii) das Einführen des Schneidwerkzeugs umfasst das Schneiden der embryonalen Achse in Längsrichtung, um ein Teil der embryonalen Achse in jedem Kotyledonensegment zu erhalten,
(iii) das Einführen des Schneidwerkzeugs umfasst das Schneiden des Sojabohnensamens in Längsrichtung entlang eines Hilum des Sojabohnensamens,
(iv) das Einführen des Schneidwerkzeugs umfasst das Vordringen des Schneidwerkzeugs in die embryonale Achse des Sojabohnensamens vor dem Ausbilden des ersten und des zweiten Kotyledonensegments, oder
(v) das Transgen ist ausgewählt aus der Gruppe bestehend aus einem Insektizidresistenzgen, Herbizidtoleranzgen, Stickstoffverwertungseffizienzgen, Wasserverwertungseffizienzgen, Nährstoffqualitätsgen, DNA-Bindegen und einem auswählbaren Markergen, wobei das Transgen bevorzugt ein *pat, dgt-28* oder *hpt* selektierbares Markergen umfasst.

3. Das Verfahren gemäß Anspruch 2 (ii), wobei die embryonale Achse des Sojabohnensamens vor dem Einführen des Schneidwerkzeugs eine ungeschnittene Länge aufweist, und der Teil der embryonalen Achse des ersten Kotyledonensegments eine Länge aufweist, die
(a) ungefähr 1/3 bis 1/2 der ungeschnittenen Länge der embryonalen Achse des Sojabohnensamens, oder
(b) gleich der ungeschnittenen Länge, ist.

4. Das Verfahren gemäß Anspruch 3 (a), außerdem umfassend das Schneiden des Anteils der embryonalen Achse des ersten Kotyledonensegments auf die Länge, die ungefähr 1/3 bis 1/2 der ungeschnittenen Länge der embryonalen Achse des Sojabohnensamens entspricht.

5. Das Verfahren gemäß Anspruch 2 (iii), außerdem umfassend das Lokalisieren des Hilums auf einer äußeren Oberfläche des Sojabohnensamens und das Anordnen des Schneidwerkzeugs in Bezug auf den Sojabohnensamen, um das Schneidwerkzeug an dem Hilum vor dem Einführen des Schneidwerkzeugs auszurichten, wobei bevorzugt jedes Kotyledonensegment einen Teil des Hilums umfasst.

6. Das Verfahren gemäß Anspruch 2 (iii), wobei der Sojabohnensamen eine Längsachse aufweist, die sich durch das Hilum erstreckt, wobei die Längsachse den Sojabohnensamen in eine erste Seite und eine zweite Seite aufteilt, wobei das Verfahren außerdem das Erfassen der ersten Seite des Sojabohnensamens vor dem Einführen des Schneidwerkzeugs umfasst.

7. Das Verfahren gemäß Anspruch 1, wobei das Verfahren außerdem das Herstellen einer vollständigen, fortpflanzungsfähigen Sojabohnenpflanze aus dem kultivierten beimpften Kotyledonensegment umfasst, wobei die vollständige, fortpflanzungsfähige Sojabohnenpflanze das transgene Ereignis enthält.

8. Ein kultiviertes, beimpftes Kotyledonensegment, hergestellt nach dem Verfahren gemäß Anspruch 1, wobei das Kotyledonensegment das transgene Ereignis enthält.

9. Ein Verfahren zum Herstellen eines transgenen Ereignisses, wobei das Verfahren umfasst:
Einführen eines Schneidwerkzeugs in eine Samenhülle eines Sojabohnensamens, um ein Kotyledonensegment zu bilden, das 1/4-, 1/3-, 1/2-, 2/3-, 3/4- oder den vollständigen Längenanteil der embryonalen Achse umfasst,
Beimpfen des Kotyledonensegments, das den Anteil der embryonalen Achse enthält mit *Agrobakterium,* wobei das *Agrobakterium* wenigstens ein Transgen enthält,
Kultivieren des beimpften Kotyledonensegments, um ein transgenes Ereignis in einer transformierten Pflanzenzelle zu bewirken, und
Herstellen einer vollständigen, fortpflanzungsfähigen Pflanze aus dem kultivierten, beimpften Kotyledonensegment, wobei die vollständige,
fortpflanzungsfähige Pflanze das transgene Ereignis enthält,
wobei der Samen bevorzugt einen Dikotyledonen-Samen darstellt.

10. Das Verfahren gemäß Anspruch 9, wobei das Einführen des Schneidwerkzeugs in die Samenhülle des Samens ein erstes Kotyledonensegment und ein zweites Kotyledonensegment bildet, wobei jedes Kotyledonensegment einen Teil der embryonalen Achse umfasst.

## Revendications

1. Procédé de production d'un évènement transgénique, le procédé comprenant les étapes consistant à :
insérer un outil de coupe dans un tégument d'une graine de soja pour former un premier segment de cotylédon et un second segment de cotylédon, la graine comprenant un axe embryonnaire ;
ensemencer le premier segment de cotylédon, comprenant un quart, un tiers, la moitié, les deux tiers, les trois quarts ou la longueur totale de l'axe embryonnaire, avec *Agrobacterium,* l'*Agrobacterium* incluant au moins un transgène ; et
cultiver le segment de cotylédon ensemencé pour produire un évènement transgénique dans une cellule végétale transformée.

2. Procédé selon la revendication 1, comprenant en outre au moins l'une des particularités suivantes :
(i) le fait d'éliminer le tégument du premier segment de cotylédon et du second segment de cotylédon avant d'ensemencer le premier segment de cotylédon,
(ii) l'insertion de l'outil de coupe comprend la coupe longitudinale de l'axe embryonnaire pour conserver une partie de l'axe embryonnaire avec chaque segment de cotylédon,
(iii) l'insertion de l'outil de coupe comprend la coupe longitudinale de la graine de soja le long d'un hile de la graine de soja,
(iv) l'insertion de l'outil de coupe comprend l'avancement de l'outil de coupe dans l'axe embryonnaire de la graine de soja avant la formation des premier et second segments de cotylédon, ou
(v) le transgène est choisi dans le groupe constitué par un gène de résistance à des insecticides, un gène de tolérance à des herbicides, un gène d'efficience de l'utilisation de l'azote, un gène d'efficience de l'utilisation de l'eau, un gène de qualité nutritionnelle, un gène de liaison à l'ADN et un gène marqueur sélectionnable, le transgène comprenant de préférence un gène marqueur sélectionnable *pat, dgt-28* ou *hpt.*

3. Procédé selon la revendication 2 (ii), dans lequel l'axe embryonnaire de la graine de soja a une longueur non coupée avant l'insertion de l'outil de coupe, et la partie de l'axe embryonnaire du premier segment de cotylédon a une longueur qui
(a) représente environ un tiers à la moitié de la longueur non coupée de l'axe embryonnaire de la graine de soja, ou
(b) est égale à la longueur non coupée.

4. Procédé selon la revendication 3 (a), comprenant en outre le fait de couper la partie de l'axe embryonnaire du premier segment de cotylédon à la longueur qui représente environ un tiers à la moitié de la longueur non coupée de l'axe embryonnaire de la graine de soja.

5. Procédé selon la revendication 2 (iii), comprenant en outre le fait de localiser le hile sur une surface externe de la graine de soja, et d'orienter l'outil de coupe par rapport à la graine de soja pour aligner l'outil de coupe avec le hile avant d'insérer l'outil de coupe, de préférence chaque segment de cotylédon incluant une partie du hile.

6. Procédé selon la revendication 2 (iii), dans lequel la graine de soja a un axe longitudinal qui s'étend à travers le hile, l'axe longitudinal divisant la graine de soja en un premier côté et un second côté, le procédé comprenant en outre le fait de saisir le premier côté de la graine de soja avant d'insérer l'outil de coupe.

7. Procédé selon la revendication 1, le procédé comprenant en outre la production d'un plant de soja entier, fertile, à partir du segment de cotylédon ensemencé, cultivé, le plant de soja entier, fertile, comprenant l'évènement transgénique.

8. Segment de cotylédon ensemencé, cultivé, produit par le procédé selon la revendication 1, dans lequel le segment de cotylédon comprend l'évènement transgénique.

9. Procédé de production d'un évènement transgénique, le procédé comprenant les étapes consistant à :
insérer un outil de coupe dans un tégument d'une graine de soja pour former un segment de cotylédon comprenant un quart, un tiers, la moitié, les deux tiers, les trois quarts ou la longueur totale de l'axe embryonnaire,
ensemencer le segment de cotylédon, comprenant la partie de l'axe embryonnaire, avec *Agrobacterium,* l'*Agrobacterium* incluant au moins un transgène,
cultiver le segment de cotylédon ensemencé pour produire un évènement transgénique dans une cellule végétale transformée, et
produire un plant entier, fertile, à partir du segment de cotylédon ensemencé, le plant entier, fertile, incluant l'évènement transgénique,
la graine étant de préférence une graine de dicotylédone.

10. Procédé selon la revendication 9, dans lequel l'insertion de l'outil de coupe dans le tégument de la graine forme un premier segment de cotylédon et un second segment de cotylédon, chaque segment de cotylédon incluant une partie de l'axe embryonnaire.


### SEQUENCE LISTING

<110> Pareddy, Dayakar
   Chennareddy, Siva
   Minnicks, Tatyana
   Karpova, Olga
   Griffin, David
   Pon Samuel, Jayakumar
   Smith, Kelley
   Sarria-Millan, Rodrigo
<120> Improved Soybean Transformation for Efficient and High-Throughput Transgenic Event Production
<130> 14764-227992 (73502)
<160> 6
<170> PatentIn version 3.5
<210> 1
   <211> 2783
   <212> DNA
   <213> Artificial sequence
<220>
   <223> YFP Plant Transcription Unit
<400> 1
   gtcgacctgc aggtcaacgg atcaggatat tcttgtttaa gatgttgaac tctatggagg      60
   tttgtatgaa ctgatgatct aggaccggat aagttccctt cttcatagcg aacttattca     120
   aagaatgttt tgtgtatcat tcttgttaca ttgttattaa tgaaaaaata ttattggtca     180
   ttggactgaa cacgagtgtt aaatatggac caggccccaa ataagatcca ttgatatatg     240
   aattaaataa caagaataaa tcgagtcacc aaaccacttg ccttttttaa cgagacttgt     300
   tcaccaactt gatacaaaag tcattatcct atgcaaatca ataatcatac aaaaatatcc     360
   aataacacta aaaaattaaa agaaatggat aatttcacaa tatgttatac gataaagaag     420
   ttacttttcc aagaaattca ctgattttat aagcccactt gcattagata aatggcaaaa     480
   aaaaacaaaa aggaaaagaa ataaagcacg aagaattcta gaaaatacga aatacgcttc     540
   aatgcagtgg gacccacggt tcaattattg ccaattttca gctccaccgt atatttaaaa     600
   aataaaacga taatgctaaa aaaatataaa tcgtaacgat cgttaaatct caacggctgg     660
   atcttatgac gaccgttaga aattgtggtt gtcgacgagt cagtaataaa cggcgtcaaa     720
   gtggttgcag ccggcacaca cgagtcgtgt ttatcaactc aaagcacaaa tacttttcct     780
   caacctaaaa ataaggcaat tagccaaaaa caactttgcg tgtaaacaac gctcaataca     840
   cgtgtcattt tattattagc tattgcttca ccgccttagc tttctcgtga cctagtcgtc     900
   ctcgtctttt cttcttcttc ttctataaaa caatacccaa agcttcttct tcacaattca     960
   gatttcaatt tctcaaaatc ttaaaaactt tctctcaatt ctctctaccg tgatcaaggt    1020
   aaatttctgt gttccttatt ctctcaaaat cttcgatttt gttttcgttc gatcccaatt     1080
   tcgtatatgt tctttggttt agattctgtt aatcttagat cgaagacgat tttctgggtt     1140
   tgatcgttag atatcatctt aattctcgat tagggtttca taaatatcat ccgatttgtt     1200
   caaataattt gagttttgtc gaataattac tcttcgattt gtgatttcta tctagatctg     1260
   gtgttagttt ctagtttgtg cgatcgaatt tgtcgattaa tctgagtttt tctgattaac     1320
   agagatctcc atgtcatctg gagcacttct ctttcatggg aagattcctt acgttgtgga     1380
   gatggaaggg aatgttgatg gccacacctt tagcatacgt gggaaaggct acggagatgc     1440
   ctcagtggga aaggtatgtt tctgcttcta cctttgatat atatataata attatcacta     1500
   attagtagta atatagtatt tcaagtattt ttttcaaaat aaaagaatgt agtatatagc     1560
   tattgctttt ctgtagttta taagtgtgta tattttaatt tataactttt ctaatatatg     1620
   accaaaacat ggtgatgtgc aggttgatgc acaattcatc tgtactaccg gagatgttcc     1680
   tgtgccttgg agcacacttg tcaccactct cacctatgga gcacagtgct ttgccaagta     1740
   tggtccagag ttgaaggact tctacaagtc ctgtatgcca gatggctatg tgcaagagcg     1800
   cacaatcacc tttgaaggag atggcaactt caagactagg gctgaagtca cctttgagaa     1860
   tgggtctgtc tacaataggg tcaaactcaa tggtcaaggc ttcaagaaag atggtcacgt     1920
   gttgggaaag aacttggagt tcaacttcac tccccactgc ctctacatct ggggagacca     1980
   agccaaccac ggtctcaagt cagccttcaa gatatgtcat gagattactg gcagcaaagg     2040
   cgacttcata gtggctgacc acacccagat gaacactccc attggtggag gtccagttca     2100
   tgttccagag tatcatcata tgtcttacca tgtgaaactt tccaaagatg tgacagacca     2160
   cagagacaac atgagcttga aagaaactgt cagagctgtt gactgtcgca agacctacct     2220
   ttgagtagtt agcttaatca cctagagctc ggtcaccagc ataattttta ttaatgtact     2280
   aaattactgt tttgttaaat gcaattttgc tttctcggga ttttaatatc aaaatctatt     2340
   tagaaataca caatattttg ttgcaggctt gctggagaat cgatctgcta tcataaaaat     2400
   tacaaaaaaa ttttatttgc ctcaattatt ttaggattgg tattaaggac gcttaaatta     2460
   tttgtcgggt cactacgcat cattgtgatt gagaagatca gcgatacgaa atattcgtag     2520
   tactatcgat aatttatttg aaaattcata agaaaagcaa acgttacatg aattgatgaa     2580
   acaatacaaa gacagataaa gccacgcaca tttaggatat tggccgagat tactgaatat     2640
   tgagtaagat cacggaattt ctgacaggag catgtcttca attcagccca aatggcagtt     2700
   gaaatactca aaccgcccca tatgcaggag cggatcattc attgtttgtt tggttgcctt     2760
   tgccaacatg ggagtccaag gtt                                             2783
<210> 2
   <211> 1828
   <212> DNA
   <213> Artificial sequence
<220>
   <223> PAT Plant Transcription Unit (PTU)
<400> 2
   ccagaaggta attatccaag atgtagcatc aagaatccaa tgtttacggg aaaaactatg        60
   gaagtattat gtaagctcag caagaagcag atcaatatgc ggcacatatg caacctatgt       120
   tcaaaaatga agaatgtaca gatacaagat cctatactgc cagaatacga agaagaatac       180
   gtagaaattg aaaaagaaga accaggcgaa gaaaagaatc ttgaagacgt aagcactgac       240
   gacaacaatg aaaagaagaa gataaggtcg gtgattgtga aagagacata gaggacacat       300
   gtaaggtgga aaatgtaagg gcggaaagta accttatcac aaaggaatct tatcccccac       360
   tacttatcct tttatatttt tccgtgtcat ttttgccctt gagttttcct atataaggaa       420
   ccaagttcgg catttgtgaa aacaagaaaa aatttggtgt aagctatttt ctttgaagta       480
   ctgaggatac aacttcagag aaatttgtaa gtttgtaggt accagatctg gatcccaaac       540
   catgtctccg gagaggagac cagttgagat taggccagct acagcagctg atatggccgc       600
   ggtttgtgat atcgttaacc attacattga gacgtctaca gtgaacttta ggacagagcc       660
   acaaacacca caagagtgga ttgatgatct agagaggttg caagatagat acccttggtt       720
   ggttgctgag gttgagggtg ttgtggctgg tattgcttac gctgggccct ggaaggctag       780
   gaacgcttac gattggacag ttgagagtac tgtttacgtg tcacataggc atcaaaggtt       840
   gggcctagga tctacattgt acacacattt gcttaagtct atggaggcgc aaggttttaa       900
   gtctgtggtt gctgttatag gccttccaaa cgatccatct gttaggttgc atgaggcttt       960
   gggatacaca gcccggggta cattgcgcgc agctggatac aagcatggtg gatggcatga      1020
   tgttggtttt tggcaaaggg attttgagtt gccagctcct ccaaggccag ttaggccagt      1080
   tacccaaatc tgagtagtta gcttaatcac ctagagctcg atcggcggca atagcttctt      1140
   agcgccatcc cgggttgatc ctatctgtgt tgaaatagtt gcggtgggca aggctctctt      1200
   tcagaaagac aggcggccaa aggaacccaa ggtgaggtgg gctatggctc tcagttcctt      1260
   gtggaagcgc ttggtctaag gtgcagaggt gttagcggga tgaagcaaaa gtgtccgatt      1320
   gtaacaagat atgttgatcc tacgtaagga tattaaagta tgtattcatc actaatataa      1380
   tcagtgtatt ccaatatgta ctacgatttc caatgtcttt attgtcgccg tatgtaatcg      1440
   gcgtcacaaa ataatccccg gtgactttct tttaatccag gatgaaataa tatgttatta      1500
   taatttttgc gatttggtcc gttataggaa ttgaagtgtg cttgaggtcg gtcgccacca      1560
   ctcccatttc ataattttac atgtatttga aaaataaaaa tttatggtat tcaatttaaa      1620
   cacgtatact tgtaaagaat gatatcttga aagaaatata gtttaaatat ttattgataa      1680
   aataacaagt caggtattat agtccaagca aaaacataaa tttattgatg caagtttaaa      1740
   ttcagaaata tttcaataac tgattatatc agctggtaca ttgccgtaga tgaaagactg      1800
   agtgcgatat tatggtgtaa tacatagg                                         1828
<210> 3
   <211> 3323
   <212> DNA
   <213> Artificial Sequence
<220>
   <223> DGT-28 Plant Transcription Unit (PTU)t
<400> 3
   gtcgacctgc aggtcaacgg atcaggatat tcttgtttaa gatgttgaac tctatggagg      60
   tttgtatgaa ctgatgatct aggaccggat aagttccctt cttcatagcg aacttattca     120
   aagaatgttt tgtgtatcat tcttgttaca ttgttattaa tgaaaaaata ttattggtca     180
   ttggactgaa cacgagtgtt aaatatggac caggccccaa ataagatcca ttgatatatg     240
   aattaaataa caagaataaa tcgagtcacc aaaccacttg ccttttttaa cgagacttgt     300
   tcaccaactt gatacaaaag tcattatcct atgcaaatca ataatcatac aaaaatatcc     360
   aataacacta aaaaattaaa agaaatggat aatttcacaa tatgttatac gataaagaag     420
   ttacttttcc aagaaattca ctgattttat aagcccactt gcattagata aatggcaaaa     480
   aaaaacaaaa aggaaaagaa ataaagcacg aagaattcta gaaaatacga aatacgcttc     540
   aatgcagtgg gacccacggt tcaattattg ccaattttca gctccaccgt atatttaaaa     600
   aataaaacga taatgctaaa aaaatataaa tcgtaacgat cgttaaatct caacggctgg     660
   atcttatgac gaccgttaga aattgtggtt gtcgacgagt cagtaataaa cggcgtcaaa     720
   gtggttgcag ccggcacaca cgagtcgtgt ttatcaactc aaagcacaaa tacttttcct     780
   caacctaaaa ataaggcaat tagccaaaaa caactttgcg tgtaaacaac gctcaataca     840
   cgtgtcattt tattattagc tattgcttca ccgccttagc tttctcgtga cctagtcgtc     900
   ctcgtctttt cttcttcttc ttctataaaa caatacccaa agcttcttct tcacaattca     960
   gatttcaatt tctcaaaatc ttaaaaactt tctctcaatt ctctctaccg tgatcaaggt    1020
   aaatttctgt gttccttatt ctctcaaaat cttcgatttt gttttcgttc gatcccaatt    1080
   tcgtatatgt tctttggttt agattctgtt aatcttagat cgaagacgat tttctgggtt    1140
   tgatcgttag atatcatctt aattctcgat tagggtttca taaatatcat ccgatttgtt    1200
   caaataattt gagttttgtc gaataattac tcttcgattt gtgatttcta tctagatctg    1260
   gtgttagttt ctagtttgtg cgatcgaatt tgtcgattaa tctgagtttt tctgattaac    1320
   agagatctcc atggcacaga tcaacaagtc tgctaatggg gttaggaacg cttcactgat    1380
   aagcaacttc tccaataccc gtcaagccaa atcccctttc tccctctcat gcggaacaag    1440
   actgaagaac agcagcagag gtttgaagaa ggtggcagtt aggctcattg gctcccgtgt 1500
   caaagtgtct gcctcagcaa gagggatgcc agccttgtct ttacctggat caaagagtat 1560
   cacagctagg gcactctttc ttgctgctgc tgctgatggg gttactactt tggtgaggcc 1620
   attgagaagt gacgacacag aaggattcgc tgaggggtta gttcgtttag gctatcgtgt 1680
   agggaggaca cccgatactt ggcaagtcga tggcagacca caaggaccag cagtggctga 1740
   ggctgacgtc tactgtagag acggagcaac caccgctaga ttcttgccaa ccttagcagc 1800
   tgctggtcac ggaacataca gatttgatgc ttcaccacag atgaggagac gtcctctttt 1860
   gcccttaagc agagccttga gggatttggg tgtcgatctt agacacgaag aagctgaagg 1920
   tcatcaccct ctgactgtcc gtgctgctgg ggttgaagga ggagaggtta ctttggatgc 1980
   tggtcagtca agtcagtatc tcactgcctt gttgctcctt ggtcccctta caagacaagg 2040
   actgaggata agggttactg atttggtgtc agcaccatac gtggagatta cgcttgcaat 2100
   gatgagggct ttcggagttg aagtggcaag ggagggagat gtgttcgttg ttccacctgg 2160
   tggatatcgt gcaactacgt atgctataga acccgacgca agtactgctt cttacttctt 2220
   cgcagctgct gctttgactc ctggagctga agtgactgta cctgggttag gcacgggagc 2280
   acttcaagga gatttgggat ttgtagatgt cttaaggaga atgggagccg aggtgtccgt 2340
   aggagctgat gcaaccactg ttagaggaac tggtgaattg cgtggcctta cagccaacat 2400
   gagagacata agtgatacga tgccgaccct cgctgcaata gcaccctttg ctagtgctcc 2460
   agttagaatc gaggatgttg ccaacactcg tgtcaaagaa tgtgacagac ttgaggcttg 2520
   tgcagagaac cttaggaggt tgggagtaag ggttgcaacg ggtccggact ggattgagat 2580
   acaccctggt ccagctactg gtgctcaagt cacaagctat ggtgatcaca gaattgtgat 2640
   gtcatttgca gtgactggac ttcgtgtgcc tgggatcagc ttcgacgacc ctggctgtgt 2700
   tcgtaagact ttccctgggt ttcacgaggc tttcgcagaa ttgaggcgtg gcattgggag 2760
   ctgagtagtt agcttaatca cctagagctc ggtcaccagc ataattttta ttaatgtact 2820
   aaattactgt tttgttaaat gcaattttgc tttctcggga ttttaatatc aaaatctatt 2880
   tagaaataca caatattttg ttgcaggctt gctggagaat cgatctgcta tcataaaaat 2940
   tacaaaaaaa ttttatttgc ctcaattatt ttaggattgg tattaaggac gcttaaatta 3000
   tttgtcgggt cactacgcat cattgtgatt gagaagatca gcgatacgaa atattcgtag 3060
   tactatcgat aatttatttg aaaattcata agaaaagcaa acgttacatg aattgatgaa 3120
   acaatacaaa gacagataaa gccacgcaca tttaggatat tggccgagat tactgaatat 3180
   tgagtaagat cacggaattt ctgacaggag catgtcttca attcagccca aatggcagtt 3240
   gaaatactca aaccgcccca tatgcaggag cggatcattc attgtttgtt tggttgcctt 3300
   tgccaacatg ggagtccaag gtt 3323
<210> 4
   <211> 1818
   <212> DNA
   <213> artificial sequence
<220>
   <223> PAT transcription unig
<400> 4
   ccagaaggta attatccaag atgtagcatc aagaatccaa tgtttacggg aaaaactatg      60
   gaagtattat gtaagctcag caagaagcag atcaatatgc ggcacatatg caacctatgt     120
   tcaaaaatga agaatgtaca gatacaagat cctatactgc cagaatacga agaagaatac     180
   gtagaaattg aaaaagaaga accaggcgaa gaaaagaatc ttgaagacgt aagcactgac     240
   gacaacaatg aaaagaagaa gataaggtcg gtgattgtga aagagacata gaggacacat     300
   gtaaggtgga aaatgtaagg gcggaaagta accttatcac aaaggaatct tatcccccac     360
   tacttatcct tttatatttt tccgtgtcat ttttgccctt gagttttcct atataaggaa     420
   ccaagttcgg catttgtgaa aacaagaaaa aatttggtgt aagctatttt ctttgaagta     480
   ctgaggatac aacttcagag aaatttgtaa gtttgtagat ctccatgtct ccggagagga     540
   gaccagttga gattaggcca gctacagcag ctgatatggc cgcggtttgt gatatcgtta     600
   accattacat tgagacgtct acagtgaact ttaggacaga gccacaaaca ccacaagagt     660
   ggattgatga tctagagagg ttgcaagata gatacccttg gttggttgct gaggttgagg     720
   gtgttgtggc tggtattgct tacgctgggc cctggaaggc taggaacgct tacgattgga     780
   cagttgagag tactgtttac gtgtcacata ggcatcaaag gttgggccta ggatccacat     840
   tgtacacaca tttgcttaag tctatggagg cgcaaggttt taagtctgtg gttgctgtta     900
   taggccttcc aaacgatcca tctgttaggt tgcatgaggc tttgggatac acagcccgtg     960
   gtacattgcg cgcagctgga tacaagcatg gtggatggca tgatgttggt ttttggcaaa     1020
   gggattttga gttgccagct cctccaaggc cagttaggcc agttacccag atctgactga    1080
   gcttgagctt atgagcttat gagcttagag ctcagatcgg cggcaatagc ttcttagcgc    1140
   catcccgggt tgatcctatc tgtgttgaaa tagttgcggt gggcaaggct ctctttcaga    1200
   aagacaggcg gccaaaggaa cccaaggtga ggtgggctat ggctctcagt tccttgtgga    1260
   agcgcttggt ctaaggtgca gaggtgttag cggggatgaa gcaaaagtgt ccgattgtaa    1320
   caagatatgt tgatcctacg taaggatatt aaagtatgta ttcatcacta atataatcag    1380
   tgtattccaa tatgtactac gatttccaat gtctttattg tcgccgtatg caatcggcgt    1440
   cacaaaataa tccccggtga ctttctttta atccaggatg aaataatatg ttattataat    1500
   ttttgcgatt tggtccgtta taggaattga agtgtgcttg cggtcgccac cactcccatt    1560
   tcataatttt acatgtattt gaaaaataaa aatttatggt attcaattta aacacgtata    1620
   cttgtaaaga atgatatctt gaaagaaata tagtttaaat atttattgat aaaataacaa    1680
   gtcaggtatt atagtccaag caaaaacata aatttattga tgcaagttta aattcagaaa    1740
   tatttcaata actgattata tcagctggta cattgccgta gatgaaagac tgagtgcgat    1800
   attatggtgt aatacata                                                  1818
<210> 5
   <211> 2135
   <212> DNA
   <213> artificial sequence
<220>
   <223> HPT Plant Transcription Unit (PTU)
<400> 5
   ccagaaggta attatccaag atgtagcatc aagaatccaa tgtttacggg aaaaactatg      60
   gaagtattat gtaagctcag caagaagcag atcaatatgc ggcacatatg caacctatgt     120
   tcaaaaatga agaatgtaca gatacaagat cctatactgc cagaatacga agaagaatac     180
   gtagaaattg aaaaagaaga accaggcgaa gaaaagaatc ttgaagacgt aagcactgac     240
   gacaacaatg aaaagaagaa gataaggtcg gtgattgtga aagagacata gaggacacat     300
   gtaaggtgga aaatgtaagg gcggaaagta accttatcac aaaggaatct tatcccccac     360
   tacttatcct tttatatttt tccgtgtcat ttttgccctt gagttttcct atataaggaa     420
   ccaagttcgg catttgtgaa aacaagaaaa aatttggtgt aagctatttt ctttgaagta     480
   ctgaggatac aacttcagag aaatttgtaa gtttgtagat ctaaacaatg aaaaagcctg     540
   aactcaccgc gacgtctgtc gagaagtttc tgatcgaaaa gttcgacagc gtctccgacc     600
   tgatgcagct ctcggagggc gaagaatctc gtgctttcag cttcgatgta ggagggcgtg     660
   gatatgtcct gcgggtaaat agctgcgccg atggtttcta caaagatcgt tatgtttatc     720
   ggcactttgc atcggccgcg ctcccgattc cggaagtgct tgacattggg gaattcagcg     780
   agagcctgac ctattgcatc tcccgccgtg ctcagggtgt cacgttgcaa gacctgcctg     840
   aaaccgaact gcccgctgtt ctgcagccgg tcgcggaggc tatggatgct atcgctgcgg     900
   ccgatcttag ccagacgagc gggttcggcc cattcggacc gcaaggaatc ggtcaataca     960
   ctacatggcg tgatttcata tgcgcgattg ctgatcccca tgtgtatcac tggcaaactg    1020
   tgatggacga caccgtcagt gcgtccgtcg cgcaggctct cgatgagctg atgctttggg    1080
   ccgaggactg ccccgaagtc cggcacctcg ttcacgcgga tttcggctcc aacaatgtcc    1140
   tgacggacaa tggccgcata acagcggtca ttgactggag cgaggcgatg ttcggggatt    1200
   cccaatacga ggtcgccaat atcttcttct ggaggccgtg gttggcttgt atggagcagc    1260
   agacgcgcta cttcgagcgg aggcatccgg agcttgcagg atcgccgcgg ctccgggcgt      1320
   atatgctccg cattggtctt gaccaactct atcagagctt ggttgacggc aatttcgatg      1380
   atgcagcttg ggcgcagggt cgatgcgacg caatcgtccg atccggagcc gggactgtcg      1440
   ggcgtacaca aatcgcccgc agaagcgcgg ccgtctggac cgatggctgt gtagaagtac      1500
   tcgccgatag tggaaaccga cgccccagca ctcgtccgag ggcaaaggaa taagtgacta      1560
   gttagcctag tcaattcact gaatcaatcg atcagtgagc tcggtcacca gcataatttt      1620
   tattaatgta ctaaattact gttttgttaa atgcaatttt gctttctcgg gattttaata      1680
   tcaaaatcta tttagaaata cacaatattt tgttgcaggc ttgctggaga atcgatctgc      1740
   tatcataaaa attacaaaaa aattttattt gcctcaatta ttttaggatt ggtattaagg      1800
   acgcttaaat tatttgtcgg gtcactacgc atcattgtga ttgagaagat cagcgatacg      1860
   aaatattcgt agtactatcg ataatttatt tgaaaattca taagaaaagc aaacgttaca      1920
   tgaattgatg aaacaataca aagacagata aagccacgca catttaggat attggccgag      1980
   attactgaat attgagtaag atcacggaat ttctgacagg agcatgtctt caattcagcc      2040
   caaatggcag ttgaaatact caaaccgccc catatgcagg agcggatcat tcattgtttg      2100
   tttggttgcc tttgccaaca tgggagtcca aggtt                                 2135
<210> 6
   <211> 1818
   <212> DNA
   <213> artificial sequence
<220>
   <223> PAT Plant Transcription Unit (PTU)
<400> 6
   ccagaaggta attatccaag atgtagcatc aagaatccaa tgtttacggg aaaaactatg      60
   gaagtattat gtaagctcag caagaagcag atcaatatgc ggcacatatg caacctatgt     120
   tcaaaaatga agaatgtaca gatacaagat cctatactgc cagaatacga agaagaatac     180
   gtagaaattg aaaaagaaga accaggcgaa gaaaagaatc ttgaagacgt aagcactgac     240
   gacaacaatg aaaagaagaa gataaggtcg gtgattgtga aagagacata gaggacacat     300
   gtaaggtgga aaatgtaagg gcggaaagta accttatcac aaaggaatct tatcccccac     360
   tacttatcct tttatatttt tccgtgtcat ttttgccctt gagttttcct atataaggaa     420
   ccaagttcgg catttgtgaa aacaagaaaa aatttggtgt aagctatttt ctttgaagta     480
   ctgaggatac aacttcagag aaatttgtaa gtttgtagat ctccatgtct ccggagagga     540
   gaccagttga gattaggcca gctacagcag ctgatatggc cgcggtttgt gatatcgtta     600
   accattacat tgagacgtct acagtgaact ttaggacaga gccacaaaca ccacaagagt     660
   ggattgatga tctagagagg ttgcaagata gatacccttg gttggttgct gaggttgagg     720
   gtgttgtggc tggtattgct tacgctgggc cctggaaggc taggaacgct tacgattgga     780
   cagttgagag tactgtttac gtgtcacata ggcatcaaag gttgggccta ggatccacat     840
   tgtacacaca tttgcttaag tctatggagg cgcaaggttt taagtctgtg gttgctgtta     900
   taggccttcc aaacgatcca tctgttaggt tgcatgaggc tttgggatac acagcccgtg     960
   gtacattgcg cgcagctgga tacaagcatg gtggatggca tgatgttggt ttttggcaaa     1020
   gggattttga gttgccagct cctccaaggc cagttaggcc agttacccag atctgactga    1080
   gcttgagctt atgagcttat gagcttagag ctcagatcgg cggcaatagc ttcttagcgc    1140
   catcccgggt tgatcctatc tgtgttgaaa tagttgcggt gggcaaggct ctctttcaga    1200
   aagacaggcg gccaaaggaa cccaaggtga ggtgggctat ggctctcagt tccttgtgga    1260
   agcgcttggt ctaaggtgca gaggtgttag cggggatgaa gcaaaagtgt ccgattgtaa    1320
   caagatatgt tgatcctacg taaggatatt aaagtatgta ttcatcacta atataatcag    1380
   tgtattccaa tatgtactac gatttccaat gtctttattg tcgccgtatg caatcggcgt    1440
   cacaaaataa tccccggtga ctttctttta atccaggatg aaataatatg ttattataat    1500
   ttttgcgatt tggtccgtta taggaattga agtgtgcttg cggtcgccac cactcccatt    1560
   tcataatttt acatgtattt gaaaaataaa aatttatggt attcaattta aacacgtata    1620
   cttgtaaaga atgatatctt gaaagaaata tagtttaaat atttattgat aaaataacaa    1680
   gtcaggtatt atagtccaag caaaaacata aatttattga tgcaagttta aattcagaaa    1740
   tatttcaata actgattata tcagctggta cattgccgta gatgaaagac tgagtgcgat    1800
   attatggtgt aatacata                                                  1818